(19) Europäisches Patentamt
European Patent Office
Office européen des brevets

(11) **EP 2 250 190 B1**

(12) **EUROPÄISCHE PATENTSCHRIFT**

(45) Veröffentlichungstag und Bekanntmachung des Hinweises auf die Patenterteilung:
**04.04.2012 Patentblatt 2012/14**

(21) Anmeldenummer: **08784517.8**

(22) Anmeldetag: **17.06.2008**

(51) Int Cl.:
*C07K 14/245* (2006.01)   *B82Y 15/00* (2011.01)
*B82Y 5/00* (2011.01)   *A61K 49/00* (2006.01)
*C07K 14/705* (2006.01)   *G01N 33/574* (2006.01)
*G01N 33/58* (2006.01)

(86) Internationale Anmeldenummer:
**PCT/EP2008/004867**

(87) Internationale Veröffentlichungsnummer:
**WO 2009/106102 (03.09.2009 Gazette 2009/36)**

(54) **OPTIMIERTE ADHÄSIN-FRAGMENTE UND ENTSPRECHENDE NANOPARTIKEL**

OPTIMIZED ADHESIN FRAGMENTS AND CORRESPONDING NANOPARTICLES

FRAGMENTS OPTIMISÉS D ADHÉSINE ET NANOPARTICULES CORRESPONDANTES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **29.02.2008 EP 08003845**

(43) Veröffentlichungstag der Anmeldung:
**17.11.2010 Patentblatt 2010/46**

(73) Patentinhaber: **Signalomics GmbH**
**1110 Wien (AT)**

(72) Erfinder:
• **BLOCK, Christoph**
**48151 Münster (DE)**
• **MITTMANN, Karin**
**48151 Münster (DE)**
• **ARNTZ, Claudia**
**49525 Lengerich (DE)**

(74) Vertreter: **von Renesse, Dorothea et al**
**König-Szynka-Tilmann-von Renesse**
**Patentanwälte Partnerschaft**
**Postfach 11 09 46**
**40509 Düsseldorf (DE)**

(56) Entgegenhaltungen:
**WO-A-2007/057182   WO-A-2008/074461**

• **BERGER CEDRIC N ET AL: "Differential recognition of members of the carcinoembryonic antigen family by Afa/Dr adhesins of diffusely adhering Escherichia coli (Afa/Dr DAEC)." MOLECULAR MICROBIOLOGY, Bd. 52, Nr. 4, Mai 2004 (2004-05), Seiten 963-983, XP002504816 ISSN: 0950-382X in der Anmeldung erwähnt**
• **GAO X ET AL: "In vivo cancer targeting and imaging with semiconductir quantum dots" NATURE BIOTECHNOLOGY, Bd. 22, Nr. 8, 18. Juli 2004 (2004-07-18), Seiten 969-976, XP002387133 ISSN: 1087-0156**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft optimierte Adhäsine und Nanopartikel, an die diese Adhäsine gebunden sind. Die Erfindung betrifft des weiteren die Bereitstellung dieser Nanopartikel als in vivo Kontrastmittel, insbesondere für die Diagnostik von Darmkrebs.

Bei einer Vielzahl von Erkrankungen ist eine möglichst frühe und aussagekräftige Diagnose von entscheidender Bedeutung für die Wahl sowie die Abstimmung und Ausführung der notwendigen medizinischen Maßnahmen. Dies gilt vor allem für eine Vielzahl von Tumorarten, für deren Bestimmung und Therapie (einschließlich möglicher Sektionen) die Diskriminierung zwischen gesundem und karzinogenem Gewebe wesentlich ist. Demnach hängt die Genesung oder sogar das Überleben eines Patienten entscheidend davon ab, ob und in welcher Güte der behandelnde und/oder operierende Arzt zwischen verschiedenen Gewebetypen unterscheiden kann.

Zur Verbesserung der Diagnose und der medizinischen Maßnahmen wurden in der Vergangenheit bereits Kontrastmittel entwickelt, mit deren Hilfe Funktionen und Strukturen im Körper über bildgebende Verfahren sichtbar gemacht werden können. Diese Verfahren werden unter anderem zur gezielten Detektion krebsassoziierter Zellveränderungen verwendet.

Aus der Wo 2007/057182 A3 sind beispielsweise Fluoreszenz-Nanopartikel bekannt, die mindestens drei Strukturen umfassen, nämlich einen anorganischen Kern, der mit einer Passivierungsschicht ummantelt wird, die ihrerseits wiederum spezifische Liganden trägt. Diese Liganden erlauben die spezifischen Bindung des Nanopartikels an ein Zielmolekül ("target"). Das Zielmolekül können vor allem spezifische Oberflächenmoleküle der Zielzellen sein, so z.B. tumorassoziierte Antigene.

Die aus dieser Druckschrift bekannten Nanopartikel mit Kern und umgebender Passivierungsschicht weisen einen kleinen hydrodynamischen Durchmesser auf, der bevorzugt zwischen 5 und 15 nm liegt. Nanopartikel dieser Größe können über die Niere ausgeschieden werden und akkumulieren daher nicht oder allenfalls nur in vertretbaren Mengen im Körper. Dies ist eine der entscheidenden Anforderungen für die Verwendung von Nanopartikeln zu medizinischen Zwecken.

Eine andere Voraussetzung ist die hohe Spezifität, mit der die an die Nanopartikel gebundenen Liganden die ausgewählte Zielstruktur im Körper erkennen. Nur eine hochspezifische Erkennung erlaubt eine Detektion, die den vornehmlichen Zweck der medizinischen Verwendung eines Kontrastmittels darstellt. Diese hochspezifischen Liganden müssen gleichzeitig so klein sein, dass die gute Verteilung und Eindringtiefe der Nanopartikel nicht behindert wird. Häufig eingesetzte spezifische Liganden wie Antikörper oder Fab-Fragmente sind in der Regel zu groß, um diese Anforderung zu erfüllen.

In den Industrieländern hat die Anzahl Darmkrebsneuerkrankungen in den letzten 30 Jahren deutlich zugenommen. Mit einer jährlichen Anzahl von Neuerkrankungen (Inzidenz) in Höhe von 30-35 pro 100.000 Einwohner ist der Darmkrebs eine der häufigsten bösartigen Erkrankungen in Mitteleuropa und ist für etwa 15 % aller Krebstodesfälle verantwortlich. Die weltweite Inzidenz wird auf eine Million Neuerkrankungen pro Jahr geschätzt. Männer sind etwas häufiger betroffen als Frauen, dies betrifft vor allem Rektumkarzinome (Geschlechtsverhältnis 60:40).

In Deutschland stellt Darmkrebs sowohl bei den Neuerkrankungen als auch beim Krebstod bei Männern wie Frauen die zweithäufigste Krebsform dar, an der 2005 mehr als 20.000 Menschen verstarben. Die Gesellschaft der epidemiologischen Krebsregister geht sogar von fast 30.000 Todesfällen pro Jahr aus.

Als Darmkrebs bezeichnet man alle bösartigen (malignen) Tumoren des Darmes. Dabei kann es sich um Karzinoide, vor allem im Wurmfortsatz und im Dünndarm, Leiomyosarkome und gastrointestinale Stromatumoren (GIST) handeln, die sich aus der glatten Muskulatur bzw. dem Bindegewebe der Darmschleimhaut ableiten. Diese Erkrankungen sind aber eher selten, und machen nur einen geringen Anteil aller Darmkrebsfälle aus. Den weitaus größte Anteil aller Darmkrebsfälle (mehr als 95%) macht das Adenokarzinom des Blinddarms (Caecum), Grimmdarms (*Colon*) oder des Enddarms (*Rectum*) aus. Dafür wird zusammenfassend auch der Begriff des kolorektalen Karzinoms (*colorectal cancer*) verwendet.

Darmkrebs, vor allem kolorektale Karzinome, verursachen zunächst sehr selten Symptome, sie entstehen fast immer aus anfangs gutartigen Darmpolypen. Die Heilungschancen durch eine Operation und anschließende Chemotherapie mit 5-Jahres-Überlebensrate von 40 bis 60 % im Mittel hängen entscheidend vom Krankheitsstadium ab, in dem der Darmkrebs entdeckt wird. Der Vorsorge und auch der frühen Diagnose kommen daher eine besondere Bedeutung zu. Letzteres wäre über eine frühzeitige Detektion von Darmkrebszellen, insbesondere CRC-Zellen (Colerectal Cancer Cells) möglich.

Zur Detektion von CRC-Zellen ist ein Target nötig, das spezifisch für diese Zellen ist und möglichst im Tumor überexprimiert wird. Für Mitglieder der CEACAM-Familie (CEA-related cell adhesion molecule; CEA = carcinoembryonic antigen) ist beschrieben, dass ihre Expression in CRC-Zellen hochreguliert ist. CEACAM-Familienmitglieder sind daher als Target zur Detektion von CRC Zellen besonders geeignet. Hierzu gehören vor allem CEA (CEACAM5) und NCA (CEACAM6).

Die CEACAM-Familie ist ein Mitglied der Ig-Superfamilie. Jedes Familienmitglied ist hochglycosyliert und besteht aus einer N-terminalen Ig-variabel-ähnlichen Domäne, an die sich bis zu 6 IgC2-Domänen anschließen. CEACAM1, CEACAM3 und CEACAM4 sind über eine carboxyterminale Transmembran- und cytoplasmatische Domäne in die Zellmembran inseriert, während CEA (CEACAM5), CEACAM6 (NCA), CEACAM7 und CEACAM8 über Glycosylphosphatidylinositol (GPI) an der Membran verankert sind. Die N-terminale Domäne weist mehr als 90 % Ähnlichkeit auf dem Aminosäurelevel innerhalb dieser Gruppe auf.

CEA und NCA sind im Colongewebe im Zylinderepithel und in den Becherzellen vorhanden. Sie befinden sich dort an der apikalen Oberfläche von reifen Enterocyten und zwar in der Glycocalyx/Microvillus Region. CEACAM agieren als interzelluläre Adhäsionsmoleküle. Diese strikte apikale Lokalisation der CEACAM in normalen Colon-Epithelzellen ist bei Adenocarcinoma-Zellen aufgehoben - die Proteine werden so auf der gesamten Zelloberfläche exprimiert. In CRC Zellen ist die Zellorganisation und die Polarität der Zellen aufgehoben, so findet man beispielsweise CEA und/oder NCA auf der gesamten Zelloberfläche lokalisiert. CEA kann freigesetzt werden, so dass es ins Blut gelangt und somit als Serum-Tumormarker detektiert

Als Liganden für diese Rezeptoren sind u.a. Adhäsine der Dr-Familie von *E. coli* Bakterien beschrieben (Afa/Dr Adhäsine, DrCEA Subfamilie). Dabei sind diese Adhäsine auf der bakteriellen Oberfläche, z. T. in den Fimbrien organisiert, von diffusely adhering *E.coli* (DAEC)-Stämmen lokalisiert und vermitteln deren Anheftung an Epithelzellen. Mitglieder dieser Familie sind z.B. AfaE-I, AfaE-III, AfaE-V, DraE und DaaE.

Die strukturellen Aufbau-Gene ("structural assembly genes"), die für Afa/Dr-Adhäsine codieren, haben eine gleichartige Organisation. Sie bestehen aus Operons, die mindestens 5 Gene (A bis E) enthalten. Dabei codieren die Gene A bis D akzessorische Gene, wobei Gen D ein Invasin codiert. Gen E codiert das eigentliche Adhäsin.

Die Gencluster haben hochkonservierte Regionen, z.B. die Gene afaA, afaB, afaC, afaD and afaF, die z.B. regulatorische oder Chaperon-Funktion haben. Das strukturelle, AfaE codierende Gen ist sehr heterogen, was zu der Produktion von antigenisch unterschiedlichen Adhäsinen führt.

*E. coli* Bakterien, die diese Mitglieder der Afa/Dr-Familie der Adhäsine exprimieren, adhärieren an CHO-Zellen, die CEA exprimieren (Berger et al.,Molecular Microbiology, (2004) 52(4), pp. 963-983, "Differential recognition of members of the carcinoembryonic antigen family by Afa/Dr adhiesins of diffusely adhering Escherichia coli (Afa/Dr DAEC)"). Adhäsine sind des Weiteren auch beschrieben bei Alain L. Servin (Clinical Microbiology Reviews (April 2005) 18(2), pp. 264-292, "Pathogenesis of Afa/Dr Diffusely Adhering Escherichia coli").

Diese bekannte Adhäsine sind aber bezüglich ihrer Affinität zu CEA noch unbefriedigend. Es ist daher Aufgabe der vorliegenden Erfindung, Adhäsine bzw. Adhäsinfragmente ("Adhäsin-Konstrukte") bereitzustellen, die eine hohe Affinität für CRC-Zellen aufweisen. Dazu sollte die Affinität zu CEA im Vergleich zu den bekannten Wildtyp-Adhäsinen möglichst verbessert sein.

Es ist außerdem Ziel der vorliegenden Erfindung, Adhäsine und/oder Adhäsin-Fragmente bereitzustellen, die eine Eignung aufweisen, als Ligand an einen für die Bereitstellung eines Kontrastmittels nutzbaren Nanopartikel gebunden zu sein. Dieser sollte vorzugsweise medizinisch, insbesondere bevorzugt als in vivo Kontrastmittel einsetzbar sein.

Zur Lösung dieser Aufgabe werden Adhäsine oder Adhäsin-Fragmente bereitgestellt, die durch eine Mutation gegenüber der jeweiligen Wildtyp-Adäsin Aminosäuresequenz, vorzugsweise der DraE Wildtyp-Sequenz, modifiziert sind. Die Modifikation führt vorteilhafterweise zu einer verbesserten Affinität der erfindungsgemäßen Adhäsine gegenüber Mitgliedern der CEACAM-Familie, insbesondere gegenüber CEA und/oder NCA. Die erfindungsgemäßen Adhäsine bzw. Adhäsin-Fragmente werden nachfolgend zusammenfassend auch als "modifizierte" oder "optimierte" Adhäsine bezeichnet.

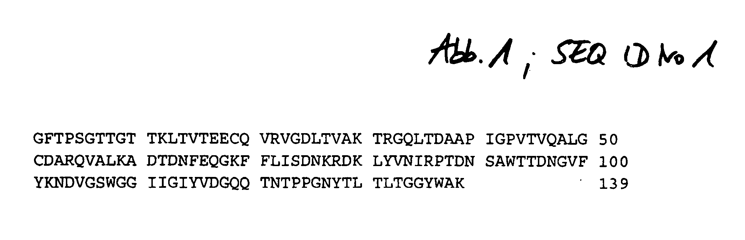

Die optimierten Adhäsine umfassen eine oder mehrere der folgenden Mutationen (Die Nummerierung der Aminosäurepositionen basiert dabei auf der DraE Wildtyp-Aminosäuresequenz; siehe auch Abb. 1).

T7(N, F, C, S, V, R, A, I, L, Y); E17 (S, P, K, G, D, R, N, H, Q); R22(T, A, S, N, K); D25(S, G, N, A, T, K, R, H, Q, M); T27(K, R, L, V, Y, P, N, Q); V28(W, F); A29 (K, R, S, E, Q, F, G, L, H, P, N, T, W); T31 (G, D, S, N); Q34(D, G, S, N, L; V, T, A); D37N; A38(S, T, L); A39(Q, S, D, M, G, F); I41(V); Q47(T, N, C, S, G, A, P); D52(G, N, S, C, P, Q, Y, H, K, R, T); N84(D, S, H); R86V; T88 (M, L); T95(L, M, Y, F, C, W, Q, N, E, S, I, H); F100(Y, V); V105(S, A, T, R, M, V, P, N, E, Q, G, K, H); I111(C, V, H, Y, T, M, F); I114(V, L, A, C); Y115(T, W, E, V); V116 (A, S, L); G118(P, S).

Sofern jedoch T88M und/oder N77K vorhanden ist, muß das erfindungsgemäße Adhäsin wenigstens eine weitere der oben genannten Mutationen aufweisen. Dies gilt insbesondere, wenn diese Mutationen in einem Adäsin der DraE-Gruppe vorliegen.

Bevorzugte Mutationen sind V28W; V28F; A39Q; A39S; I41L; Q47S; Q47T; I85L; T95L; G118S und T123I.

Im Sinne der Erfindung wird als "Adhäsin" grundsätzlich ein jedes Protein bezeichnet, das an die N-terminale Domäne eines Rezeptor aus der CEACAM Familie binden kann (nachfolgend auch Bindungsprotein), insbesondere an CEA und/oder NCA. Dazu gehören Adhäsine der sog. Adhäsin-Gruppe, der sog. DraE-Gruppe sowie insbesondere die Proteine DraE sowie AfaE-III. Dazu gehören ebenso Proteine mit den aus diesen Adhäsinen abgeleiteten Konsensussequenzen.

Die Adhäsine nach der Erfindung weisen vorzugsweise eine Sequenz von 139 Aminosäuren und eine Größe von 17 KDa auf. Sie können jedoch auch N-terminal und/oder C-terminal deletiert sein, so lange damit die Bindungsfähigkeit an CEACAM, insbesondere an CEA und/oder NCA, nicht verloren geht. Die C-terminale Deletion sollte möglichst 5, 8 oder aber maximal 10 Aminosäuren nicht überschreiten. N-terminal ist eine Deletion von bis zu 18 Aminosäuren möglich. Es sind aber auch kürze beliebige Deletionen, beispielsweise von 5, 8 oder 10 Aminosäuren möglich. In einer Ausführungsform der Erfindung weisen die Adhäsine daher eine Länge von 111 Aminosäuren auf.

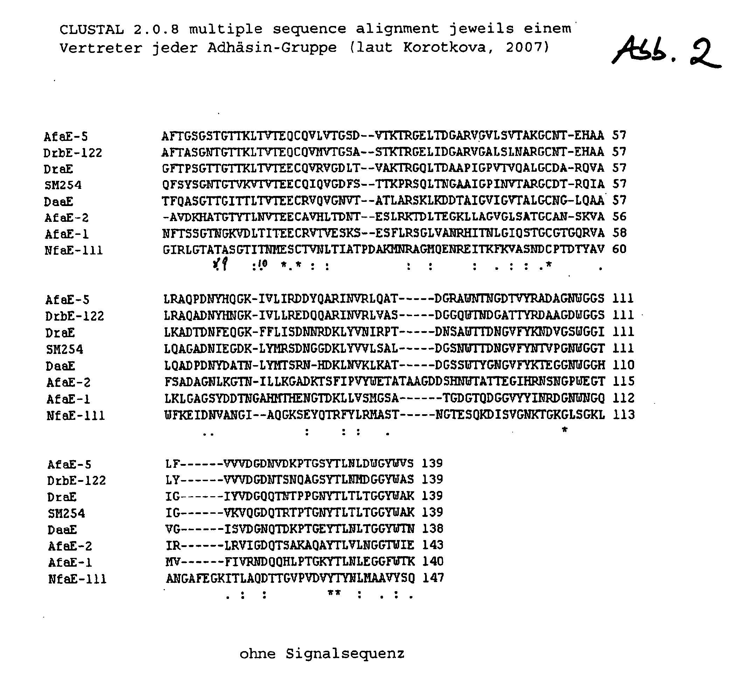

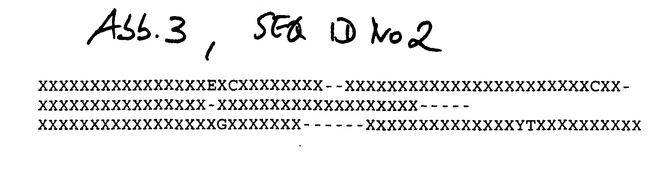

Die sog. "Adhäsin-Gruppe" besteht aus AfaE-5, DrbE-122, DraE, SM254, DaaE; AfaE-2, AfaE-1 sowie NfaE-111 (siehe dazu auch Abb. 2; Alignment). Sie weisen erfindungsgemäß eine oder mehrere der oben genannten Mutationen auf.

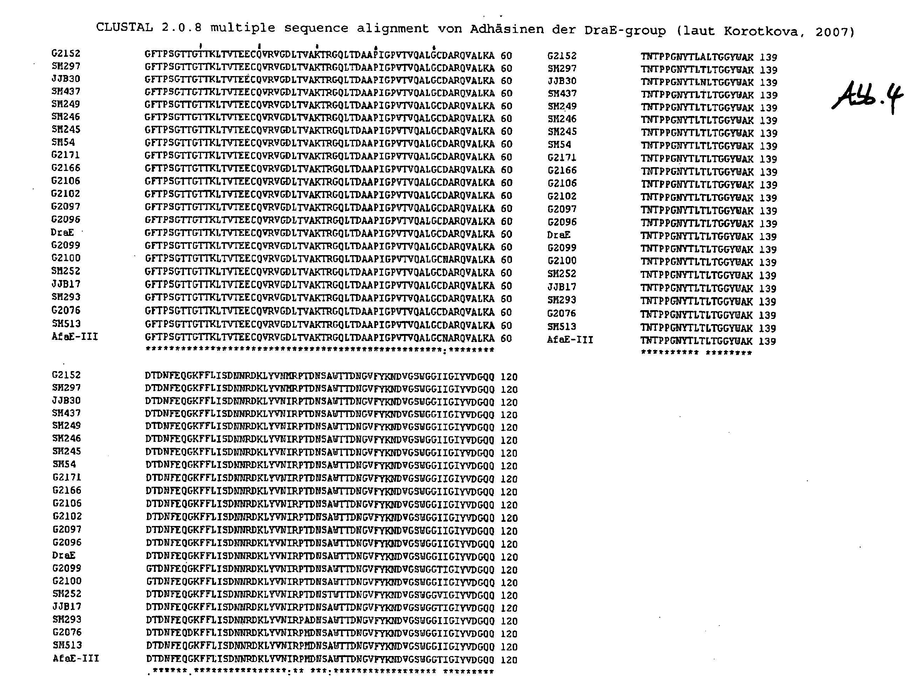

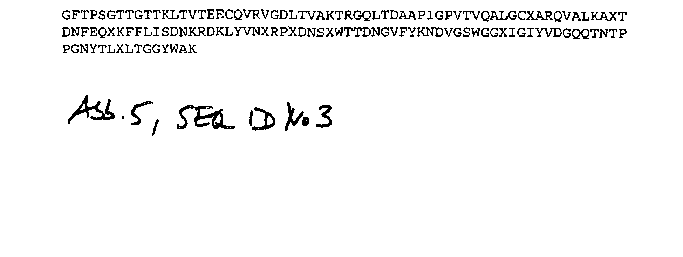

Gleiches gilt es für die sog. DraE-Gruppe, die aus den folgenden Proteinen besteht (siehe dazu auch Abb. 4, Alignment): G2152, SM297, JJB30, SM437, SM249, SM246, SM245, SM54, G2171, G2166, G2106, G2102, G2097, G2096, DraE, G2099, G2100, SM252, JJB17, SM293, G2076, SM513, AFaE-III. Diese weisen einen Konsensus nach Abb. 5 auf. In einer Ausführungsform betrifft die Erfindung demnach Adhäsine mit der Sequenz der Abb. 5 mit einer oder mehreren der oben genannten Mutationen.

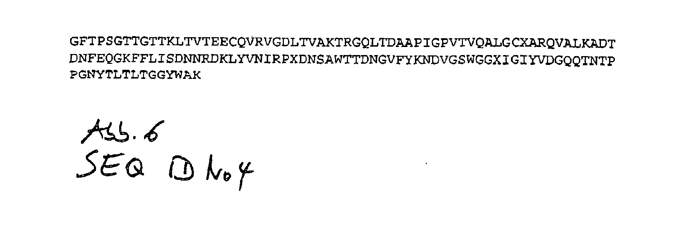

Besonders bevorzugt sind Adhäsine mit einem Konsensus nach Abb. 6. Dieser leitet sich aus dem Alignment von DraE und AfaE-III ab. Besonders bevorzugt ist DraE.

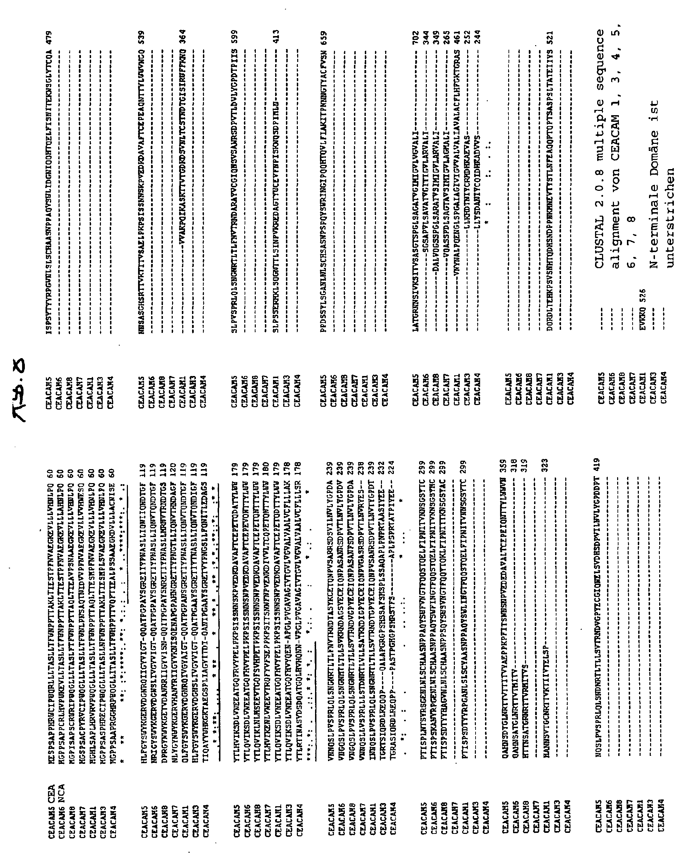

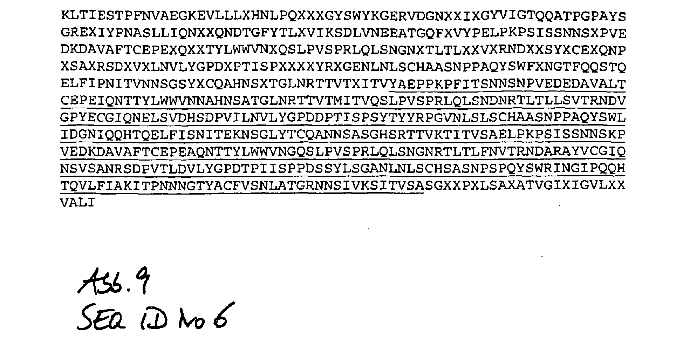

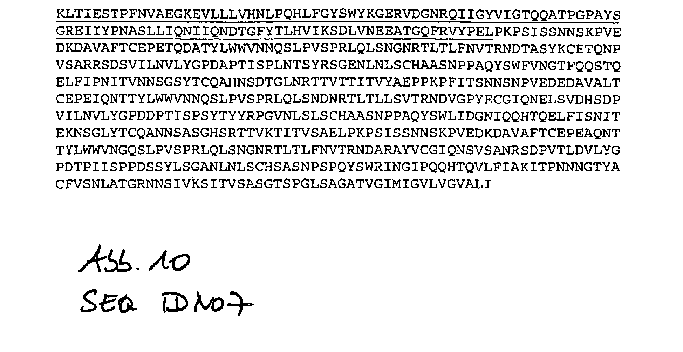

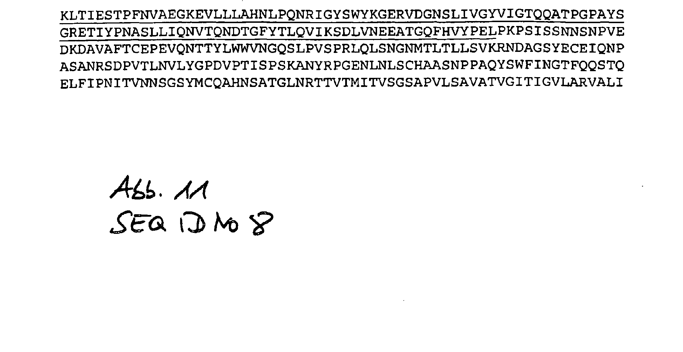

Die erfindungsgemäßen Adhäsine binden an die N-terminale Sequenz von Proteinen der CEACAM-Gruppe. Mitglieder dieser Gruppe sind CEA (CEACAM 5), NCA (CEACAM 6), CEACAM 1,3,4,7 und 8 (siehe auch Abb. 8). Bevorzugt binden sie an die N-terminale Sequenz von Proteinen mit der Sequenz der Abb. 9, die aus dem Alignment von CEA und NCA abgeleitet ist (Abb. 9, unterstrichen ist der Bereich, in dem nur CEA vorhanden ist). In einer Ausführungsform binden die erfindungsgemäßen Proteine an die N-terminale Domäne von CEA (Abb. 10) und NCA (Abb. 11).

In einer bevorzugten Ausführungsform handelt es sich bei den modifizierten Adhäsinen nach DraE (Sequenz in Abb. 1), die an CEA und/oder NCA binden mit einer der oben genannten

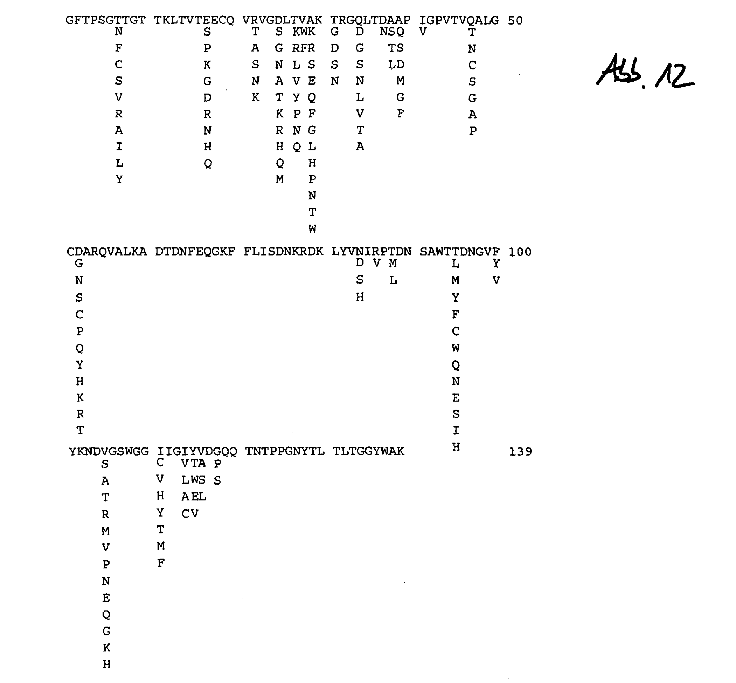

Mutationen. Bevorzugt ist somit ein Adhäsin mit einer Konsensussequenz nach Alignment Abb. 12 wie folgt:

XXXXXX T7(N, F, C, S, V, R, A, I, L, Y) XXXXXXXXX E17 (S, P, K, G, D, R, N, H, Q) XXXX R22(T, A, S, N, K) XX D25(S, G, N, A, T, K, R, H, Q, M) X T27(K, R, L, V, Y, P, N, Q) V28(W, F) A29(K, R, S, E, Q, F, G, L, H, P, N, T, W) X T31(G, D, S, N) XX Q34(D, G, S, N, L, V, T, A) XX D37N A38(S, T, L) X A39(Q, S, D, M, G, F) X 141 (V) XXXXX Q47(T, N, C, S, G, A, P) XXXX D52(G, N, S, C, P, Q, Y, H, K, R, T) XXXXXXXXXXXXXXXXXXXXXXXXXXXXX N84(D, S, H) X R86V X T88(M, L) XXXXXX T95(L, M, Y, F, C, W, Q, N, E, S, I, H) XXXX F100(Y, V) XXXX V105(S, A, T, R, M, V, P, N, E, Q, G, K, H) XXXXX I111(C, V, H, Y, T, M, F) XX I114(V, L, A, C) Y115(T, W, E, V) V116(A, S, L) X G118(P, S) XXXXXXXXXXXXXXXXXXXX

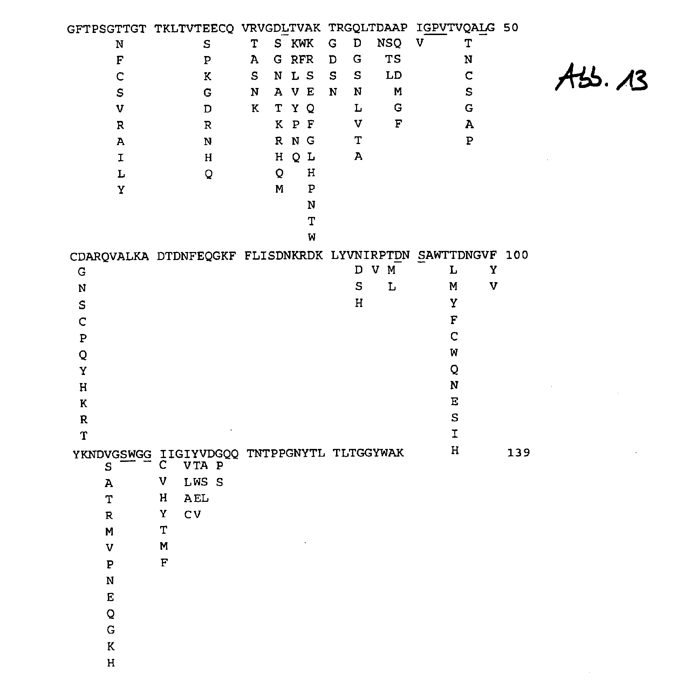

In einer weiteren Ausführungsform weisen die Adäsine, insbesondere aus DraE eine Konsensussequenz nach Alignment in Abb. 13):

XXXXXX T7(N, F, C, S, V, R, A, I, L, Y) XXXXXXXXX E17 (S, P, K, G, D, R, N, H, Q) XXXX R22(T, A, S, N, K) XX D25(S, G, N, A, T, K, R, H, Q, M) L26 T27 (K, R, L, V, Y, P, N, Q) V28(W, F) A29(K, R, S, E, Q, F, G, L, H, P, N, T, W) X T31 (G, D, S, N) XX Q34 (D, G, S, N, L, V, T, A) XX D37N A38(S, T, L) X A39 (Q, S, D, M, G, F) X I41(V) G42 P43 V44 XX Q47(T, N, C, S, G, A, P) X L49 XX D52(G, N, S, C, P, Q, Y, H, K, R, T) XXXXXXXXXXXXXXXXXXXXXXXXXX XXXX N84(D, S, H) X R86V X T88(M, L) D89 X S91 XXX T95(L, M, Y, F, C, W, Q, N, E, S, I, H) XXXX F100(Y, V) XXXX V105(S, A, T, R, M, V, P, N, E, Q, G, K, H) X S107 W108 X G110 I111(C, V, H, Y, T, M, F) XX I114(V, L, A, C) Y115(T, W, E, V) V116(A, S, L) X G118(P, S) XXXXXXXXXXXXXXXXXXXX

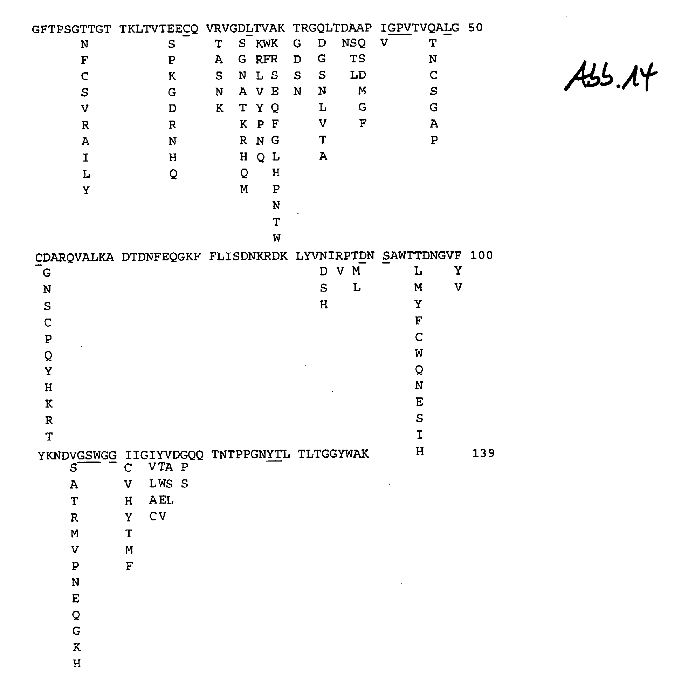

In einer anderen Ausführungsform weisen die Adhäsine, insbesondere aus DraE folgende Konsensussequenz auf (Abb. 14):

XXXXXX T7(N, F, C, S, V, R, A, I, L, Y) XXXXXXXXX E17 (S, P, K, G, D, R, N, H, Q) X C19 XX R22(T, A, S, N, K) XX D25(S, G, N, A, T, K, R, H, Q, M) L26 T27(K, R, L, V, Y, P, N, Q) V28(W, F) A29(K, R, S, E, Q, F, G, L, H, P, N, T, W) X T31 (G, D, S, N) XX Q34(D, G, S, N, L, V, T, A) XX D37N A38(S, T, L) A39(Q, S, D, M, G, F) X 141 (V) G42 P43 V44 XX Q47(T, N, C, S, G, A, P) X L49 X C51 D52(G, N, S, C, P, Q, Y, H, K, R, T) XXXXXXXXXXXXXXXXXXX XXXXXXXXXXX N84(D, S, H) X R86V X T88(M, L) D89 X S91 XXX T95(L, M, Y, F, C, W, Q, N, E, S, I, H) XXXX F100(Y, V) XXXX V105(S, A, T, R, M, V, P, N, E, Q, G, K, H) G106 S107 W108 X G110 1111 (C, V, H, Y, T, M, F) XX I114(V, L, A, C) Y115(T, W, E, V) V116(A, S, L) X G118(P, S) XXXXXXXXX Y128 T129 XXXXXXXXXX

Die genannten Konsensussequenzen aus den Abb. 12 bis 14 basieren auf der Nummerierung der Sequenz in Abb. 1. Auch hier können die oben erwähnten Deletionen am N-Terminus und/oder C-Terminus vorhanden sein, solange die Bindung des Adhäsins an ein CEACAM-Mitglied, insbesondere CEA und/oder NCA nicht unmöglich wird.

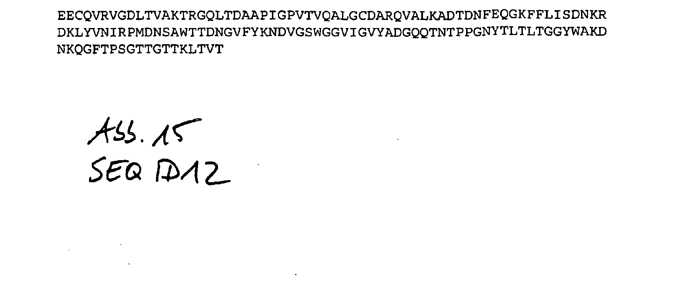

In einer besonderen Ausführungsform ist ein Adhäsin der Sequenz in Abb. 1 bevorzugt, das die folgenden Mutationen umfasst, nämlich N77K, T88M, I111V, I114V und V116A (nachfolgend auch "Fünffach-Mutante, FM; Abb.15).

Dem Fachmann ist grundsätzlich bekannt, Adhäsin-Monomere herzustellen. So wird beispielsweise in dem rekombinanten Protein AfaE-III-dsc der N-terminale vernetzende Abschnitt G an den C-Terminus kloniert sowie ein Linker zwischen F und G eingefügt. Der Abschnitt G faltet sich dann über den Linker zurück und bindet an den jetzt N-terminalen Abschnitt A1 und stabilisiert dadurch die monomere Struktur. Durch Gelfiltrationen kann diese monomere Struktur des AfaE-III-dsc nachgewiesen werden (s. Abb. 1C, aus Anderson et al., 2004).

Eine weitere Möglichkeit zur Erzeugung monomerer Adhäsine beschreiben Piatek et al. (Infection and Immunity (2005) 73(1) pp. 135-145, "Molecular Aspects of Biogenesis of Escherichia coli Dr Fimbriae: Characterization of DraB-DraE-Complexes"). Sie entfernen unterschiedlich viele Aminosäuren vom N-Terminus des Adhäsins und erhalten so monomere Proteine (s. Abb. 2, aus Piatek et al., 2005). Diese bekannten Prinzipien sind dem Grunde nach auch für die erfindungsgemäß modifizierten Adhäsine anwendbar.

Um ein modifiziertes Adhäsin zu erhalten, kann ein Wildtyp Adhäsin einer Mutagenese unterzogen werden, zum Beispiel einer Oligonukleotid-basierten, ortsspezifischen Mutagenese (z.B. EP1777292 A1).

Zur Herstellung der erfindungsgemäßen Adhäsine kann in einer bevorzugten Ausführungsform ein Adhäsin-Konstrukt mit einer verringerten Affinität zu DAF (Decay Accelerating Factor, CD55) als Template für die weitere(n) Mutagenese(n) eingesetzt werden. Ein Beispiel dafür ist die Mutation N77K (van Loy et al., Molecular Microbiology (2002) 45(2), pp. 439-452, "Identification of amino acids in Dr adhesin required for binding to decay-accelerating factor").

Die durch die Mutagenese entstehenden Banken können zusammen mit dem Target in Hefe transformiert und der Reportergen-Readout detektiert werden (siehe dazu auch die Ausführungsbeispiele unten). Auf diesem Weg können die erfindungsgemäßen Adhäsine mit einer verbesserten Affinität zu CEA und/oder NCA identifiziert werden.

Die erfindungsgemäßen Adhäsine können an Nanopartikel gebunden sein. Diese können vorzugsweise als in vivo Kontrastmittel für die Detektion von CRC Zellen eingesetzt werden. Vorteilhafte Nanopartikel sind aus der WO2007/057182 A3 bekannt. Diese wird zu Zwecken der Offenbarung der Herstellung sowie Verwendung der Nanopartikel vollumfänglich in Bezug genommen. Dabei handelt es sich insbesondere um Nanopartikel, deren hydrodynamischer Durchmesser 15 nm nicht überschreitet, und die in biologischen Systemen nicht-inert sind.

Die erfindungsgemäßen Nanopartikel umfassen mindestens drei Strukturen, nämlich einen anorganischen Kern, der mit einer Passivierungsschicht ummantelt wird, die dann ihrerseits spezifische Liganden trägt, wobei die spezifischen Liganden auch Teil der Passivierungsschicht sein können. Diese führen zu der spezifischen Bindung des Nanopartikels an das Zielmolekül ("target") des biologischen Systems.

Bei den bevorzugten Nanopartikeln weist der anorganische Kern mit der ihn umgebenden Passivierungsschicht einen hydrodynamischen Durchmesser von möglichst nicht mehr als 15, vorzugsweise nicht mehr als 10 nm auf. Besonders bevorzugt sind hydrodynamische Durchmesser von nicht mehr als 8 nm oder nicht mehr als 5 nm. Dies gilt vor allem für sphärische Nanopartikel. Nanopartikel dieser Größe können über die Niere ausgeschieden werden und akkumulieren daher nicht oder allenfalls in vertretbaren Mengen im Körper. Damit wird die in vivo Anwendung ermöglicht. Dies gilt insbesondere für Nanopartikel mit einem hydrodynamischen Durchmesser von nicht mehr als 5 nm.

In einer alternativen Ausführungsform können die Nanopartikel auch stäbchenförmig sein. In dieser Ausgestaltung ist es von Vorteil, wenn der Durchmesser des Stäbchens die oben genannte Grenze von 15 nm nicht überschreitet. Auch hier sind Durchmesser im Bereich von 5, 8 oder 10 nm bevorzugt, um die Ausscheidung aus dem Körper zu erleichtern. So können die erfindungsgemäß einsetzbaren Nanopartikel beispielsweise eine Längen/Breitenausdehnung von 8 x 15 nm aufweisen.

Die nach der Erfindung einsetzbaren Nanopartikel emittieren vorzugsweise eine Wellenlänge mit einem Emissionmaximum zwischen 600 und 700 nm, beispielsweise zwischen 620 und 660, besonders bevorzugt von etwa 625 nm oder 655 nm. Diese Emission ist für das menschliche Auge gut sichtbar, und solche Nanopartikel können daher bei medizinischen Eingriffen unmittelbar als Kontrastmittel dienen. Demnach ist unter Umständen ein Verzicht auf unterstützende optische Instrumente möglich.

In einer alternativen Ausführungsform können Nanopartikel erfindungsgemäß eingesetzt werden, die die oben genannten hydrodynamischen Durchmesser überschreiten, sofern sichergestellt ist, dass die Partikel in vivo nicht-inert sind. Dies ist nämlich die Voraussetzung dafür, dass diese Partikel biologisch degradierbar und damit die darin zunächst partikulär gebundenen Metalle (z.B. Cd) in die Ionenform überführt werden. Die Degradationsprodukte sind über die Niere ausscheidbar.

Wie sich bereits erwiesen hat, sind anorganische Nanopartikel mit einer Passivierungsschicht enthaltend eine Imidazolkomponente in vivo tatsächlich nicht-inert, sondern werden unter diesen Bedingungen abgebaut. Somit erfüllen diese Nanopartikel das für die in vivo Anwendung besonders relevante Kriterium biologischer Degradierbarkeit und der Nierengängigkeit der Degradationsprodukte. Diese Erkenntnis war überraschend, weil die Passivierungsschicht vor allem auch der Erhöhung der chemischen und/oder physikalischen Stabilität der Nanopartikel dient (siehe dazu auch die weiteren Ausführungen unten). Somit steht die Stabilität der Nanopartikel einerseits, die für eine gute Diagnostik erforderlich ist, und die Biodegradierbarkeit andererseits, die für die Nierengängigkeit "großer" Partikel erforderlich ist, in einem für die Verwendung als in vivo Kontrastmittel geeigneten Verhältnis zueinander.

Der Passivierungsschicht kommt vor allem die Aufgabe zu, die Fluoreszenzintensität sowie die chemische und physikalische Stabilität des anorganischen Kerns zu erhöhen. Die mit der Passivierungsschicht ummantelten anorganischen Kerne sind gekennzeichnet durch eine Quantenausbeute von mindestens 10 %, vorteilhafterweise mindestens 30, 50 oder sogar 70 %. Als Quantenausbeute wird dabei das Verhältnis der Menge des von einer Probe emittierten Lichts zu der Menge des von der Probe absorbierten Lichts verstanden. Die Passivierungsschicht weist vorteilhafterweise eine Dicke von nicht mehr als 1 nm auf. Der Durchmesser des passivierten Kerns erhöht sich in diesem Fall um nicht mehr als 2 nm.

Vorteilhafterweise werden die Nanopartikel jeweils noch mit Modifikatoren, insbesondere zur Verbesserung der Kompatibilität mit der biologischen Umgebung versehen. Die Zunahme des hydrodynamischen Radius durch die Verwendung von Modifikatoren überschreitet vorzugsweise 2 nm nicht. Die Dicke der Passivierungsschicht und der Modifikatoren hängt im Einzelfall auch von den Verhältnissen beider Strukturen untereinander und im Verhältnis auf den anorganischen Kern ab.

Im Falle der oben genannten Größenbeschränkung der erfindungsgemäßen Nanopartikel weisen sie eine besondere Eignung zur Verwendung als Diagnostikum im lebenden Patienten auf. So wird durch die Größenreduktion eine Erhöhung der Diffusionsgeschwindigkeit und der Eindringtiefe in das Gewebe erreicht. Dies erlaubt eine gleichmäßige und schnelle Ausbreitung der Nanopartikel in der biologischen Umgebung sowie eine möglichst weitgehende Durchdringung eines Gewebes (z.B. eines Tumors) nach einer lokalen Applikation. Ebenso erlauben die erfindungsgemäßen Nanopartikel eine systemische Applikation, die auch über eine Injektion erfolgen kann. Die lokale Applikation, z.B. eine topische Applikation oder eine intra- bzw. peritumorale Applikation bei der Behandlung von Tumoren ist aber auch möglich.

Besonders vorteilhafte Ausführungsformen der Erfindung mit den modifizierten Adhäsinen gekoppelten Nanopartikel weisen einen hydrodynamischen Durchmesser von nicht mehr als 8, besonders bevorzugt von nicht mehr als 4 nm auf. Nanopartikel dieser Größenordnung können bereits über die Niere ausgeschieden werden, so dass sie nicht oder deutlich weniger im Körper akkumulieren. Somit vermindern die erfindungsgemäßen Nanopartikel das mit den bekannten Quantum Dots voraussichtlich verbundene Problem der Langzeittoxizität erheblich.

Die Nanopartikel emittieren vorteilhafterweise ein Fluoreszenzspektrum zwischen 600 und 700 nm, besonders bevorzugt mit einem Emissionsmaximum zwischen 600 bis 660 nm, insbesondere bevorzugt zwischen 620 bis 660 nm. Dieses Emmissionspektrum hat den Vorteil der möglichst hohen Gewebetransmission aufgrund einer nur geringen Absorption durch Hämoglobin und anderer lichtabsorbierende Substanzen im lebenden System (einschließlich Wasser). Licht dieser Wellenlängen ist für das menschliche Auge noch wahrnehmbar, so dass die Identifizierung des markierten Gewebes durch den behandelnden Arzt ohne weitere aufwendige technische Hilfsmittel zur Detektion (z.B. CCD-Kameras) möglich ist. Dies ist insbesondere bei der Verwendung der erfindungsgemäßen Nanopartikel als Kontrastmittel während eines chirurgischen Eingriffs zur Identifikation der CEA und/oder NCA exprimierenden Zellen, insbesondere zur Diskriminierung des karzinogenen und gesunden Gewebes vorteilhaft.

In einer Ausführungsform handelt es sich bei den bevorzugt einsetzbaren Nanopartikel um bekannte Nanopartikel mit einem Kern beispielsweise aus CdSe, CdS oder CdTe, wie sie z.B. in der US 2004/0247861 unter Bezugnahme auf wissenschaftliche Publikationen beschrieben werden. In dieser Druckschrift wird auch auf Dokumente zur Herstellung der Kernmaterialien verwiesen, so z.B. auf die US 6,179,912. Diese Dokumente werden zur Offenbarung der Eigenschaften dieser bekannten Nanopartikel sowie deren Herstellung vollumfänglich in Bezug genommen. Ein Verfahren zur Herstellung von Nanopartikeln ist des weiteren auch aus der US 7,147,712 B2 bekannt. Auch diese wird zu Zwecken der Offenbarung in Bezug genommen.

Es ist von besonderem Vorteil, wenn der anorganische Kern der Nanopartikel im wesentlichen aus Halbleitern besteht. Diese Kerne emittieren je nach ihrer individuellen Größe und/oder Zusammensetzung Licht in verschiedenen Farben, absorbieren aber alle breitbandig im selben Bereich des Lichtspektrums (UV bis VIS Bereich). Die Anregungs- und Emissionsspektren liegen aufgrund des hohen "Stokes shift" weit auseinander, was eine einfache und gleichzeitige Anregung verschiedener Nanopartikel ermöglicht. Sie weisen schmale und symmetrische Emissionsspektren auf, die sich nicht bzw. nur geringfügig überlappen. Weitere positive Eigenschaften, die insbesondere für die verbesserte Eindringtiefe und die *in vivo* Markierung von großer Bedeutung sind, stellen die hohe Quantenausbeute von bis zu 80% und die hohe Photostabilität dar.

Bevorzugte Nanopartikel sind z.B. aus der WO2005/001889 bekannt. Danach handelt es sich um einen anorganischen Kern aus einer Legierung aus mindestens zwei Halbleitern, die entweder homogen verteilt sind oder aber für die innerhalb der Legierung jeweils ein Konzentrationsgradient vorliegt. Hinsichtlich der Offenbarung der Beschaffenheit und der Herstellung dieser Nanopartikel wird auf oben zitierte WO2005/001889 verwiesen. Die Kerne können in ihrer Größe um jeweils 5 % abweichen.

Demnach kann der anorganische Kern der Nanopartikel eine Legierung aus mindestens zwei Halbleitern umfassen, wobei der Kern eine homogene Zusammensetzung aufweist und durch eine "Band-Gap-Energie" charakterisiert ist, die nicht-linear zu dem molaren Verhältnis der zwei Halbleiter ist.

Alternativ kann der Kern nicht-homogen beschaffen sein, wobei die Konzentration des ersten Halbleiters graduell ansteigt ausgehend vom Zentrum des Kerns bis zur Oberfläche des Kerns und die Konzentration des zweiten Halbleiters graduell abnimmt vom Zentrum des Kerns bis zu dessen Oberfläche.

Es gilt für beide Kerne gleichermaßen, dass mindestens einer der Halbleiter ein Gruppe II-Gruppe VI-Halbleiter oder ein Gruppe III-Gruppe V-Halbleiter ist (die Gruppendefinition korrespondiert mit den Gruppen des Periodensystems der Elemente). Die Legierung kann beispielsweise ausgewählt sein aus der Gruppe folgender Legierungen: CdSeTe, CdSSe, CdSTe, ZnSeTe, Zn-CdTe, CdHgS, CdHgTe, InGaAs, InGaP, GaAlAs, In-GaN. Diese Kerne können zudem eine Beschichtung aus anorganischem Material wie z.B. Halbleitern (z.B. ZnS) tragen. Diese zusätzliche Schicht ist dem Fachmann als "capping" oder "shell" bekannt.

Gruppe II-Gruppe VI und Gruppe III-Gruppe V Halbleiter sind allgemein bekannt und beinhalten z.B. $CdS_{1-x}Se_x$, $CdS_{1-x}Te_x$, $CdSe_{1-x}Te_x$, $ZnSe_{1-x}Te_x$, $Zn_{1-x}Cd_xTe$, $Cd_{1-x}Hg_xS$, $Cd_{1-x}Hg_xTe$, $In_{1-x}Ga_xAs$, $Ga_{1-x}Al_xAs$ und $In_{1-x}Ga_xP$. Vorzugsweise werden die Halbleiter $CdSe_{1-x}Te_x$, $CdS_{1-x}Te_x$, $ZnSe_{1-x}Te_x$, $Zn_{1-x}Cd_xTe$, $Cd_{1-x}Hg_xS$, $Cd_{1-x}Hg_xTe$, $In_{1-x}Ga_xAs$, $In_{1-x}Ga_xP$ verwendet, wobei x eine Fraktion von 0 bis 1 ist.

Das molare Verhältnis der Halbleiter kann jedes molare Verhältnis annehmen. Allerdings ist in dem Fall, dass die Legierung CdSSe umfasst, eine Legierung mit der molekularen Formulierung $CdS_{1-x}Se_x$ bevorzugt. In dem Fall, dass die Legierung CdSTe umfasst, ist eine Legierung mit der molekularen Formulierung $CdS_{1-x}Te_x$ bevorzugt. In dem Fall, dass die Legierung ZnSeTe umfasst, ist eine Legierung mit der molekularen Formulierung $ZnSe_{1-x}Te_x$ bevorzugt. In dem Fall, dass die Legierung ZnCdTe umfasst, ist eine Legierung mit der molekularen Formulierung allein aus CdTe bevorzugt. In diesen Angaben ist x jeweils eine Fraktion zwischen 0 und 1.

Diese bevorzugten anorganischen Kerne der Nanopartikel können mit folgenden Schritten hergestellt werden: (i) die Herstellung einer ersten Lösung unter Bedingungen, die die Bildung von Nanokristallen ermöglichen, (ii) Herstellung einer zweiten Lösung, die eine Vorstufe der Halbleiter umfasst mit einem molaren Verhältnis unter einer Bedingung, welche keine Bildung von Nanokristallen ermöglicht, (iii) Zugabe der zweiten Lösung zur ersten Lösung, die eine Bildung von Nanopartikeln ermöglicht, und (iv) Veränderung der Bedingungen, die das Wachstum der Nanokristalle und ihrer Bildung anhalten/ stoppen. Das Verfahren zur Herstellung der Kerne wird in der WO 2005/001889 dargestellt, auf die hinsichtlich der Offenbarung der Herstellung dieser bevorzugten Ausführungsform des anorganischen Kerns der erfindungsgemäßen Nanopartikel Bezug genommen wird.

In einer alternativen Ausführungsform kann der anorganische Kern im Wesentlichen aus einem Edelmetallcluster bestehen, der vorzugsweise 2 und 27 Edelmetall-Atome umfasst. In einer bevorzugten Ausführungsform wurde das Edelmetall aus einer Gruppe bestehend aus Gold, Silber, Kupfer, Platin, Palladium, Osmium, Iridium, Ruthenium und Rhodium ausgewählt. Das Cluster kann variierende Ladungen aufweisen.

Diese Kerne haben den Vorteil, dass sie aufgrund ihrer starken Absorption und Emission leicht als einzelne sogenannte "Nanodots" mit einer schwachen Quecksilber-Lampen-Anregung detektierbar sind. Die erfindungsgemäßen Nanopartikel mit diesen Kernen sind vorteilhaft als fluoreszentes Einzelmolekül- und Massenlabel zu verwenden.

Der Begriff "Edelmetall" auf eine Elementengruppe ausgewählt aus einer Gruppe bestehend aus Gold, Silber und Kupfer und der Gruppe der Platinmetalle (PGM) Platin, Palladium, Osmium, Iridium, Ruthenium und Rhodium. In bevorzugten Ausführungsformen der vorliegenden Erfindung werden die Edelmetalle aus der Gruppe bestehend aus Gold, Silber und Kupfer ausgewählt. In einer besonders bevorzugten Ausführungsform ist das Edelmetall Silber oder Gold.

Der Begriff "Cluster" bezieht sich auf eine Verbindung von 2-27 Atomen eines Metalls. Cluster sind u. a. aus den Bereichen der chemische Katalyse, der Keramik, der Halbleitertechnologie und der Materialwissenschaften bekannt. Ihre Herstellung ist dem Fachmann daher geläufig. Die WO2004/003558 beschreibt u.a. die Herstellung von Edelmetallclustern und enthält darüber hinaus umfangreiche weiterführende Literaturangaben dazu. Insbesondere ist die Herstellung von Edelmetall-Nanoclustern offenbart, die mit organischen Molekülen assoziiert sind. Der Begriff Assoziation ist dabei zu verstehen als jede Form der Bindung unabhängig von der chemischen oder physikalischen Natur der Bindung (so z.B. kovalente, nicht-kovalente, elektrostatische oder van-der-Waals-Bindung). Hinsichtlich der Herstellung der Nanocluster als Kern der erfindungsgemäßen Nanopartikel wird auf die WO2004/00355 in Bezug genommen.

Die erfindungsgemäß bevorzugt einsetzbaren Nanopartikel weisen eine Passivierungsschicht auf, die die Fluoreszenzintensität erhöht und die chemische und physikalische Stabilität des anorganischen Kerns verbessert. Damit emittieren die Nanopartikel Licht vorzugsweise mit einer Quantenausbeute von mehr als 10 %, vorzugsweise von mehr als 50 %.

Diese Nanopartikel weisen in wässriger Umgebung bei 4°C vorzugsweise eine Lagerstabilität von mindestens 12 Monaten auf und sind möglichst über einen pH-Bereich von pH 5 bis pH 10, vorzugsweise von pH 7 bis pH 10 stabil, d.h., sie zeigen Abweichungen von weniger als 50 % in Bezug auf ihre spezifischen spektralen Charakteristika wie Quantenausbeute, Lage des Emissionsmaximums, Halbwertsbreite des Emissionsspektrums. Bevorzugte Partikel zeigen Abweichungen von weniger als 10 % in Bezug auf diese spezifischen spektralen Charakteristika.

Die gemäß einer weiteren Ausführungsform der Erfindung einsetzbaren Nanopartikel zeigen auch unter biologischen (d.h. physiologischen) Bedingungen bzw. *in vivo* für einen Zeitraum von mindestens drei Tagen im wesentlichen eine Konstanz/Stabilität der Eigenschaften des Kerns (einschließlich der ihn umgebenden Passivierungsschicht). Bevorzugte Partikel zeigen eine derartige Konstanz/Stabilität für einen Zeitraum von 7 bis 14 Tagen, wobei als Stabilität das Beibehalten von mindestens 50% der eine Konstanz der Eigenschaften. Diese Angaben beziehen sich vor allem auf die Stabilität der Nanopartikel in dem eigentlichen Zielorgan. Beachtlich ist, dass die Stabilität der Nanopartikel in Organen, die primär dem Abbau dienen, deutlich weniger stabil sein können (so z.B. in der Leber). Dies kann sogar ausdrücklich gewollt sein.

Obwohl die Nanopartikel im obigen Sinne stabil sind, sind sie gleichwohl *in vivo* grundsätzlich abbaubar und demnach nicht-inert. In diesem Sinne bedeutet "nicht-inert", dass die Nanopartikel nach einem Zeitraum von 12 Wochen oder mehr nach der Administration bereits zu mindestens 50 % abgebaut sind. Vorzugsweise ist ein mindestens 50%iger Abbau bereits nach 8, 6 oder 4 Wochen nachweisbar. Der Nachweis der im Körper verbliebenen Partikel schließt dazu den Nachweis in Körperorganen sowie im Plasma ein. "Inert" heißt demnach, dass nach einem Zeitraum von 4 Wochen nach der Administration noch mehr als 50%, sogar bis nahezu 100% der Partikel im Körper des Patienten nachweisbar sind.

Die Abbaubarkeit der Nanopartikel ist über Assays nachweisbar, die dem Fachmann bekannt sind, nämlich zum Beispiel durch induktiv gekoppelte Plasma-Massenspektroskopie (ICP-MS), die bei geeigneter Probenbeschaffenheit auch durch fluoreszenzspektroskopische Messungen ergänzt werden können (siehe dazu auch unten).

Die Passivierungsschicht enthält mindestens eine Verbindung, die in der Lage ist, Metallatome oder Metallionen, z.B. Zink-, Quecksilber- oder Cadmiumionen zu koordinieren. Diese Verbindung kann eine Lewis-Base oder eine cyclisch oder linear ungesättigte Verbindung mit resonanten Elektronen sein. Als cyclisch ungesättigte Verbindung kann sie auch ein Heterozyklus bzw. ein Heteroaromat sein. Die ungesättigte oder konjugierte Gruppe befindet sich in einer bevorzugten Ausführungsform in einer terminalen Position bezogen auf die Struktur des Moleküls. Weiterhin kann die Passivierungsschicht einen Quervernetzer aufweisen oder die cyclisch oder linear ungesättigte Verbindung kann auch als Quervernetzer fungieren. Der Quervernetzer kann basisch sein.

Die Metallatome oder Metallionen koordinierenden Verbindungen können funktional durch Chelatisierung, Koordination oder Elektronen-Donor-Eigenschaften von Lewis-Basen an fluoreszente anorganische Kerne binden und entsprechend konjugierte Anteile/ Gruppen aufweisen. Diese Moleküle können zudem Anteile enthalten, die den Kernen, die mit ihnen beschichtet sind, in wässrigen Lösungen Löslichkeit oder Benetzbarkeit vermitteln.

Diese Moleküle oder Verbindungen können ein homogenes oder heterogenes (heterocyclisches) Ringsystem mit ein, zwei oder mehr gebundenen (oder auch kondensierten) Ringen beinhalten. Bevorzugte heteroaromatische Systeme sind z.B. Thiazole, Thiazolderivate, Oxazole, Oxazolderivate, Pyrrole, Pyrrolderivate einschließlich dotierter oder nicht-dotierter Polypyrrol-Oligomere, Thiophene, Thiophenderivate einschließlich dotierter und nicht-dotierter Polythiophene, Furane, Furanderivate, Pyridin und -derivate, Pyrimidin und seine Derivate, Pyrazine, Pyrazinderivate, Triazin und -derivate, Triazole, Triazolderivate, Phthalocyanine und -derivate, Porphyrin und Porphyrinderivate. Diese Verbindungen können ungesättigte (olefinische) Kohlenwasserstoffe oder deren Amine, Phosphor- oder Sauerstoffderivate beinhalten, die Acetylen, Propin und Allen mit einschließen können, aber nicht auf diese begrenzt sind. Es ist zu bevorzugen, dass das Molekül eine ausreichende p oder pi-Elektronen-Dichte aufweist, um sich an der Adduktbildung oder der Resonanz auf der Oberfläche des Halbleiter-Kerns zu beteiligen.

Die heteroaromatische Verbindung ist vorzugsweise eine Imidazol-Komponente. Vorzugsweise wird weiterhin als Quervernetzer eine Phosphin-Verbindung, vorzugsweise eine Alkylphosphin-Verbindung zugesetzt.

Mit dem Begriff "Imidazol-Komponente" wird im Sinne dieser Beschreibung ein heterozyklisches oder heteroaromatisches Molekül verstanden, das mindestens eine Imidazol-Gruppe (einschließlich Imidazol-Derivate) enthält, und das für die Bindung des anorganischen Kerns oder der Passivierungsschicht mit einem Metall wie Cadmium, Zink, Gallium oder einem Metallkation oder einem Substrat, das ein solches Kation enthält, zur Verfügung steht. In diesem Zusammenhang sollte vorzugsweise mindestens eine Imidazol-Gruppe in einer terminalen Position bezogen auf die Struktur des Moleküls sein. Die Imidazol-Komponente bindet in ihrer funktionellen Form über den Ring, der delokalisierte Molekülorbitale enthält, an den fluoreszenten Nanokristall. Gewöhnlich dienen die Stickstoffe des Imidazolrings als koordinierende Liganden, um in funktioneller Weise ein Metall-Ion wie Cadmium oder Zink zu binden.

In einer Ausführungsform umfasst die Imidazol-Komponente reaktive funktionelle Gruppen wie eine oder zwei Aminosäure(n), z.B. Histidin, Carnosin, Anserin, Balein, Homocarnosin, Histidylphenylalanin, cyklo-Histidylphenylalanin, 5-amino-4-Imidazolcarboxamid, Histidylleucin, 2-Mercaptoimidazol, boc-Histidin, Hydrazid, Histinol, 1-Methylhistidin, 3-Methylhistidin, Imidazolysin, Imidazol-enthaltendes Ornithin (z. B. 5-Methylimidazol), Imidazol-enthaltendes Alanin (z. B. (beta)-(2-Imidazolyl)-L(alpha) Alanin), Carzinin, Histamin. Diese auf Histidin basierenden Moleküle oder Imidazol-haltige Aminosäuren können nach allgemein bekannten Methoden synthetisiert werden.

Unter dem Begriff "Phosphin" wird im Sinne der Erfindung ein Molekül verstanden, das mindestens eine Phosphingruppe (einschließlich ihrer Derivate) für die Bindung oder das Chelatisieren eines Nichtmetalls wie Se, S oder anderer Nichtmetalle aufweist oder von Substraten, die solche Atome enthalten und das mindestens eine funktionelle Gruppe (z.B. Hydroxyl-, Amino-, Thiol-, Carboxyl-, Carboxamid- etc.) zur Reaktion mit benachbarten Molekülen bereitgestellt.

Vorzugsweise sollte mindestens eine Phosphin-Gruppierung in einer terminalen Position bezogen auf die Struktur des Moleküls lokalisiert sein. Die Phosphin-Anteile dienen als koordinierende Liganden, um in funktionaler Form mit einem fluoreszenten Kern oder einer Verbindung aus der abschirmenden Schicht ein Nichtmetall oder Ion wie Se oder S zu binden.

In einer bevorzugten Ausführungsform beinhaltet die Phosphin-haltige Verbindung eine, zwei oder mehrere miteinander (z.B. in polymerer Form) gekoppelte Phosphin-Gruppen, die Hydroxymethylphosphin-Verbindungen oder ähnliches mit einschließen können, aber nicht auf diese begrenzt sind. Phosphin-enthaltende Verbindungen können nach allgemein bekannten Methoden synthetisiert werden. Wie weiterhin bekannt ist, können Alkylphosphinhaltige Verbindungen außerdem eine oder mehrere zusätzliche funktionelle Gruppen (z.B. Hydroxyl-, Amino-, Thiol-, Carboxyl-, Carboxamid- etc.) aufweisen. Beispiele für Derivate sind Hydroxymethlyphosphinderivate, Amide oder Ester, sofern die Derivatisierung mit den hier beschriebenen Funktionen des Phosphins als Coating vereinbar ist.

Besonders bevorzugt sind Tris-(hydroxymethyl-)phosphin und β-[Tris-(hydroxymethyl)phosphino-]propansäure, um die fluoreszenten anorganischen Kerne der erfindungsgemäßen Nanopartikel zu beschichten. Allgemein bekannt ist, dass quervernetzte Phosphin-haltige Verbindungen zusätzlich die Möglichkeit haben, funktional an Metallatom und/oder -ionen wie Zn oder Cd zu binden. In dieser Hinsicht funktionalisierte Isocyanate oder Alkylcyanoacrylate können weiterhin als Quervernetzer für Liganden und Adduktbildung mit fluoreszenten Kernen nützlich sein. Die Quervernetzer können auch basisch sein.

Dem passivierenden Effekt der erfindungsgemäß vorhandenen Passivierungsschicht liegt das Abschirmen von oberflächlichen Cadmium- oder Zinkatomen oder ähnlichem durch die Komplexierung mit dem Heteroaromaten oder Heterozyklus (vorzugsweise mit der Imidazol-Komponente) und das Abschirmen der Gegenatome (Se oder S oder Ähnliches) über die Komplexierung mit den Phosphin-haltigen Verbindungen zugrunde.

Die Passivierungsschicht der erfindungsgemäßen Nanopartikel ist aus der US 2004/0247861 A1 bekannt. In dieser Offenlegungsschrift wird die Herstellung von mit der Passivierungsschicht ummantelten anorganischen Kernen, zum Beispiel von Quantum Dots beschrieben. Zu Zwecken der Offenbarung der Herstellung der erfindungsgemäß eingesetzte Passivierungsschicht und der damit ummantelten anorganischen Kerne wird die US 2004/0247861 daher in Bezug genommen.

Die Moleküle der Passivierungsschicht können weiterhin chemische Gruppen aufweisen oder tragen, um Zielmoleküle und Zellen zu binden und querzuvernetzen (spezifische Liganden). In der Gegenwart entsprechend passender Reagenzien wie $ZnSO_4$ und $Na_2S$ können diese Moleküle oder Verbindungen mit den Molekülen auf dem fluoreszenten Kern eine Passivierungsschicht bilden ("capping" oder "shell"). Diese Reagentien können auch an Atome oder Ionen auf der Oberfläche des fluoreszenten Nanokristalls funktional binden, so dass diese zusätzliche Passivierungsschicht auch unmittelbar auf der Oberfläche des Kerns ausgebildet sein kann.

Die erfindungsgemäßen Nanopartikel können in einer vorteilhaften Ausführungsform zusätzlich Modifikatoren aufweisen, die aus organischen und/oder anorganischen Anteilen bestehen können. Sie dienen der verbesserten Kompatibilität, Effektivität und/oder Löslichkeit der Nanopartikel in einer Flüssigkeit oder einem Suspensionsmedium insbesondere in der physiologischen Umgebung. Diese Oberflächenmodifikation ist vor allem vorteilhaft, um eine möglichst geringe unspezifische Adsorption und eine erhöhte Verträglichkeit in biologischen Systemen, insbesondere im menschlichen Körper, zu erreichen.

Eine Möglichkeit ist die Modifikation der Oberfläche mit dem für bestimmte medizinische Anwendungen bereits zugelassenen Polyethylenglykol (PEG), insbesondere in niedermolekularen Formen, um eine geringe Gesamtgröße des Nanopartikels beizubehalten. Dies kann sowohl die Biokompatibilität der Nanopartikel sowie deren Blutzirkulationszeit und auch die Aufnahmeeffizienz in Zellen erhöhen. Durch die Kombination einer niedermolekularen PEG-Schicht mit weiteren Substanzen wie Vitaminen wie z.B. Folsäure, kann eine geringere Aufnahme der Nanopartikel in Makrophagen erzielt werden, da aufgrund der damit reduzierten Proteinadsorption an den Nanopartikeln eine Erkennung der Nanopartikel durch das Immunsystem erschwert wird.

Die Beschichtung mit Monosacchariden, Di- oder Trisacchariden bis hin zu niedermolekularen Polysacchariden aus einem oder verschiedenen Monosacchariden stellt eine weitere Möglichkeit der vorteilhaften Oberflächenmodifikation durch Verwendung von Modifikatoren dar. Als eine Ausführungsart ist eine Modifikation mit z.B. Polyglucose möglich, in der Dextran eingesetzt werden kann, welches als Blutersatzstoff medizinisch zugelassen ist. Es zeigt eine gute Biokompatibilität/Verträglichkeit. Eine weitere Ausführungsform ist die Verwendung von stereoisomeren Formen (D-/L-) der Saccharide, um einer möglichen Degradation entgegenzuwirken.

Eine weitere Ausführungsform ist die Verwendung von biologisch kompatiblen hydrophilen Vitaminen als Modifikatoren wie z.B. Thiamin, Riboflavin, Niacin, Pyridoxin, Cobalamin, Panthothensäure, Ascorbinsäure und Folsäure. So kann z.B. Folsäure zu einer bevorzugten Bindung von Nanopartikeln an Krebszellen führen. Dieses Vitamin zeigt nur eine geringe Immunogenität und somit eine hohe Biokompatibilität. Durch die Bindung an den membranständigen Folsäurerezeptor wird eine Internalisierung der Nanopartikel erleichtert.

Die Oberflächenmodifikation mit lipophilen Vitaminen wie Retinol, Cholecalciferol, Tocopherol und Phyllochinon ist ebenso möglich. So kann z.B. Vitamin E zu einer erhöhten zellulären Aufnahme von Nanopartikeln führen.

Fettsäuren, wie z.B. 1-Oktadecene oder 18-Methyleicosanoidsäure und deren Derivate, können die Löslichkeit und Stabilität der Kolloide erhöhen und verfügen über eine terminale funktionelle Carboxylgruppe, die zur nachfolgenden Bindung spezifischer Liganden genutzt werden kann. Daher ist es sinnvoll, Fettsäuren als Modifikatoren mit aufzunehmen.

Eine weitere Ausführungsform der Oberflächenmodifikation ist eine Beschichtung mit Polyalkoholen, wie z.B. Diethylenglykol (DEG), die besonders gut unspezifische Proteinadsorption reduzieren können. Gleiches gilt für Polytetrafluoroethylen (PTFE, Teflon), insbesondere in seinen niedermolekularen Formen, aufgrund derer eine reduzierte Proteinadsorption erreicht werden kann. Polytetrafluoroethylen wird häufig in kardiochirurgischen Applikationen eingesetzt.

Eine Oberflächenmodifikation kann ebenfalls mit einer oder mehreren natürlich vorkommenden Aminosäuren, zu denen sowohl die proteinogenen als auch nicht-proteinogenen Aminosäuren zählen, sowie synthetischen Aminosäuren vorgenommen werden. Dabei können beide Stereoisomere (D- und L-Formen) verwendet werden. Di-, Tri-, Tetra- bis hin zu kleinen Polypeptiden aus den oben genannten Aminosäuren stimulieren kaum das Immunsystem und sind somit ebenfalls für eine dünne Kompatibilitätsschicht geeignet. Dabei kann es sich um künstliche Aminosäuresequenzen als auch Sequenzen aus biologischen Proteinen handeln. Peptidabkömmlinge von natürlichen Proteinen wie z.B. des Phytochelatins können ebenfalls zur Oberflächenmodifikation verwendet werden. Eine Oberflächenmodifikation mit Tat-Peptid und Tat-Peptid enthaltenden Peptiden ist eine weitere Möglichkeit, Nanopartikel für den Einsatz in biomedizinischen Applikationen verfügbar zu machen. Das Tat-Peptid ist ein effektives Molekül, um z.B. Goldnanopartikel durch die Zellmembran bis in den Kern zu bringen.

Eine weitere Ausführungsform der möglichen Modifikatoren ist die Ausbildung einer Phosphorylcholin-Beschichtung. Phosphorylcholin reduziert eine mögliche unspezifische Proteinadsorption, wie z.B. auf Kontaktlinsen. Eine Phophorylcholin-Modifikation kann aufgrund der nicht-thrombogenen Eigenschaften gut in biologischen Systemen eingesetzt werden und zeichnet sich durch eine hohe Langzeitstabilität aus.

Da Polylactat biokompatibel ist, wird diese Substanz in vielfältigen medizinischen Anwendungen eingesetzt. Insbesondere niedermolekulare Formen des Polylactats sind eine weitere Möglichkeit der Oberflächenmodifikation der erfindungsgemäßen Nanopartikel. Dabei können beide Stereoisomere (D-/L-Form) eingesetzt werden, um eine mögliche Biodegradation zu reduzieren.

Neben den genannten Oberflächenmodifikationen ist eine proteolytisch spaltbare Bindung von unspezifischen Proteinen an die Nanopartikel möglich. Dies kann eine Erhöhung der Biokompatibilität/Verträglichkeit bewirken. Am Zielort kann eine Abspaltung des großen Proteins erfolgen unter Freisetzung der kleinen Nanopartikel im Gewebe. Ebenso kann die Abspaltung nach entsprechender Verweildauer erfolgen. Dazu eignen sich bevorzugt weit verbreitete Proteine wie z.B. Transferrin, Lactoferrin, Ceruloplasmin, Elastin und Albumin neben anderen Proteinen, die eine unspezifische Adsorption reduzieren. So kann z.B. eine Oberflächenbeschichtung aus Kombinationen von Polypeptiden mit Elastin unerwünschte Thrombenbildung verhindern und somit die Biokompatibilität der Nanopartikel erhöhen.

Das Hauptserumprotein Albumin kann nichtspezifische Interaktionen mit Plasmamembranen reduzieren. Des weiteren behält das entsprechend modifizierte Nanopartikel die Fähigkeit, spezifische Interaktionen mit Zielzellen durch gleichzeitige Bindung eines spezifischen Liganden an der Partikeloberfläche auszubilden. Eine Beschichtung mit Serumalbumin kann zu einer wesentlich längeren Blutzirkulationszeit durch die Verhinderung einer schnellen Aufnahme durch Makrophagen nach intravenöser Gabe führen als dies bei unbeschichteten Nanopartikeln der Fall ist.

Neben den oben skizzierten unspezifischen Beschichtungen tragen die erfindungsgemäßen Nanopartikel eine selektive Markierung mit Zielzell-spezifischen Liganden, beispielsweise sind sie konjugiert mit Proteinen, Antikörpern, Peptiden oder, besonders bevorzugt, mit kleinen, hochaffinen Proteindomänen, Antikörperfragmenten oder anderen organischen Molekülen, die z.B. an Tumorzell-spezifische Strukturen oder andere Targets binden. Ein bevorzugter Ligand ist Adhäsin (siehe Ausführungsbeispiel 4). "Spezifisch" bedeutet in diesem Zusammenhang, dass der Ligand entweder ausschließlich oder aber in erhöhtem Maße auf dem Target exprimiert ist.

Die Kombination aus reduziertem hydrodynamischen Durchmesser, der zu der erwähnten höheren Diffusions- und Perfusionsgeschwindigkeit führt, zusammen mit den bereits beschriebenen Eigenschaften und Verbesserungen und der hohen Fluoreszenzintensität vor allem im sichtbaren roten Bereich des Lichts macht die erfindungsgemäßen Nanopartikel zu einem einfachen, vielfältig einsetzbaren Diagnostikum für eine selektive und genaue Diskriminierung von Gewebeformen *in vivo*. Diese Möglichkeiten in Kombination mit antigenspezifischen Biomarkern dienen vor allem der Identifikation von CEA und/oder NCA exprimierenden Zellen, insbesondere der Unterscheidung von abnormem, (prä)karzinogenem von normalem Gewebe, was während eines operativen Eingriffes eine visuelle Beurteilung zur präziseren Tumorresektion unterstützt. Die hierbei einsetzbaren erfindungsgemäßen Nanopartikel dienen somit als Kontrastmittel. Dies gilt insbesondere für die Anwendung bei der Darmkrebs-Diagnose und Operation.

Die Nanopartikel mit den erfindungsgemäßen modifizierten Adhäsinen können entweder als *in vitro* oder *in vivo* Diagnostikum, Theranostikum und oder Therapeutikum eingesetzt werden. Dazu können sie lokal (z.B. intratumoral, intramuskulös oder in operativ zugängliche Gewebe/Organe) oder aber auch systemisch (z.B. intravenös) verabreicht werden. Die lokale/topische Verabreichung kann als Flüssigkeit, Sprühlösung, Gel, Schaum, Creme, Wirkpflaster vorgesehen sein. Dies kann insbesondere bei der Behandlung/der Diagnose von Hohlorganen wie beim Darmkrebs bevorzugt sein. Auch ist eine orale Einnahme z.B. als Saft oder in Form von Tabletten oder Kapseln möglich. Gleichermaßen ist eine Inhalation (z.B. Spray) möglich. Eine anale Applikation ist über Zäpfchen vorgesehen. In einer Variante können die Nanopartikel in Depotform implantiert werden.

Der Begriff "Diagnostikum" wird im Kontext der vorliegenden Erfindung als Synonym zu "Kontrastmittel" verwendet, d.h. es dient der diskriminierenden Darstellung morphologischer oder funktioneller Strukturen in biologischen Systemen, insbesondere im lebenden Menschen, zur Unterstützung eines medizinischen Eingriffs.

Die Nanopartikel können als Diagnostikum vor allem bei chirurgischen Eingriffen eingesetzt werden. Ebenso können sie bei minimal-invasiven Verfahren (z.B. Endoskopie, Laparoskopie) verwendet werden. Sinnvoll ist eine Kombination mit bildgebenden Verfahren wie PET, MRT, CT etc.

Wie bereits oben ausgeführt, ist die erfindungsgemäße Verwendung in der Form der lokalen Applikation von besonderem Vorteil. Die bei der lokalen Applikation eingesetzte Cd-Menge überschreitet dabei vorteilhafterweise nicht ein Zehntel der Gesamtbelastung, die im Laufe des Lebens üblicherweise in der Leber und Niere eines Menschen in fortgeschrittenem Lebensalter und gewöhnlicher Lebensweise ohnehin akkumulieren. Die Gesamtbelastung dieser Organe liegt bei etwa 18 mg (Saturag et al 2000; "British Journal of Nutrition; 2000, (84), 791-802). Vorteilhafterweise wird demnach bei der lokalen Applikation die Menge an Nanopartikeln so begrenzt, dass die zugeführte Cd-Menge den Wert von 2 mg zumindest nicht wesentlich überschreitet. In einer besonders bevorzugten Ausführungsform wird die Tumorvisualisierung bereits mit einer Menge an Kontrastmittel möglich, die eine Gesamtmenge von 0,6 mg, besonders bevorzugt 0,2 mg Cadmium nicht überschreitet.

Als "lokale Applikation" wird im Sinne der Erfindung jede Applikation verstanden, bei der bereits nach Art und Weise der Applikation eine erhöhte Menge oder Dosis des Kontrastmittels in distinkten Bereichen des Körpers zu erwarten ist. Demnach ist auch eine vaskuläre Administration des Kontrastmittels eine lokale Administration, wenn durch Begleitmaßnahmen des verabreichenden Personals, wie beispielsweise das Abbinden von zu- oder abführenden Gefäßen, verhindert wird, dass sich das Kontrastmittel über das Blutgefäßsystem im Körper im wesentlichen ungehindert verteilt.

Der besondere Vorteil dieser Ausführungsform liegt darin, dass damit die Verwendung der Nanopartikel in der medizinischen Anwendung am lebenden Menschen erst möglich wird, da diese anderenfalls - d.h. als systemischen Gabe - aufgrund der damit verbundenen Toxizität ausgeschlossen ist. Die lokale Applikation reduziert nämlich die für die ausreichende Darstellung erforderliche Dosis der Nanopartikel.

Es hat sich herausgestellt, dass das Cd-haltige Kontrastmittel erfindungsgemäß zur Visualisierung eines Tumors *in vivo* vorteilhafterweise in einer Dosis eingesetzt wird, die einer Menge von 0,002 bis 0,02 mg Cd pro $cm^3$ Tumorgewebe entspricht. Besonders vorteilhaft sind Dosierungen des Kontrastmittels von 0,002 bis 0,015 mg Cd/$cm^3$ Tumorgewebe, insbesondere zwischen 0,002 und 0,010 mg Cd/$cm^3$. Mit dieser vorteilhaften Dosierung lassen sich Tumoren mit einem Volumen bis zu etwa 150 $cm^3$ *in vivo* visualisieren, ohne damit die für den Menschen üblicherweise akzeptable Belastungsobergrenze zu überschreiten. Die Visualisierung von Tumoren mit einem Volumen von bis zu 50 $cm^3$ ist besondern günstig.

Gegenstand der Untersuchungen können alle zugänglichen Gewebe/Organe des Patienten sein, vor allem die Haut, Hohlorgane (z.B. im Gastrointestinal-, Urogenital-, Respirationstrakt) oder auch äußerlich zugängliche Bereiche der Sinnesorgane und auch das kardiovaskuläre System.

Auch ist der Einsatz als *in vitro* Diagnostikum möglich, so z.B. die Immunhistochemie oder FACS sowie ELISA. Besonders vorteilhaft ist eine Kombination von *in vivo* und in vitro Diagnostik (z.B. Biopsie-Material).

Die erfindungsgemäßen modifizierten Adhäsine können an den Nanopartikel gebunden bleiben oder abspaltbar bzw. ablösbar oder freisetzbar sein.

## AUSFÜHRUNGSBEISPIELE

### I. Bereitstellung der modifizierten Adhäsine

Für die Bindung von Adhäsinen reicht die N-terminale Domäne der CEACAM, z. B. von CEA, aus. Dies ist aus Bindungsstudien mit gereinigten Fimbrien, in denen auch das jeweilige Adhäsin vorhanden ist, bekannt (Korotkova et al., The Journal of Biological Chemistry (2006) 281(39), pp. 29120-29139, "A Subfamily of Dr Adhesins of Escherichia coli Bind Independently to Decay-accelerating Factor and the N-domain of Carcinoembryonic Antigen"). Daher wurde als Target für die Adhäsine gemäß der vorliegenden Ausführungsform die N-terminale Domäne von CEA und NCA (CEACAM6) eingesetzt.

Dazu wurde zuvor die Codon Usage der N-terminalen Domäne von CEA und NCA sowie des reifen Proteins von DraE als Ligand (Adhäsin) für die Expression in *Saccharomyces cerevisiae* optimiert. Die Gene wurden synthetisiert.

DraE-Konstrukte, vorzugsweise mit der Mutation N77K, und die terminale Domäne von CEA (nachfolgend N-CEA) wurden in TH-Vektoren der Hefe kloniert und in Hefe transformiert.

Das Adhäsin wird einer Mutagenese unterzogen, in einer besonders bevorzugten Ausführungsform einer Oligo-basierten, ortsspezifischen Mutagenese (z.B. EP1777292 A1). Die daraus resultierende Bank wird durch Sequenzierung von Einzelkolonien und der Bank-DNA charakterisiert.

Die Bank sowie das Target (N-CEA) werden in den durch Integration des Met1-Gens (codiert für eine Uroporphyrinogen III Methyltranferase) in das Genom veränderten Hefestamm Y190 transformiert und die Hefen auf Medium mit 25 mM 3-AT ausplattiert. Damit die Reportergene Met1 und ß-Galaktosidase abgelesen werden, müssen die beiden Proteine interagieren.

Die Durchführung des quantitativen Screenings erfolgt wie in der EP1721974 A1 beschrieben. Das Reportergen Met1 wird durch die aus einer enzymatischen Umsetzung von Uroporphyrinogen III resultierenden Fluoreszenz des Produktes nachgewiesen.

Zur Bestätigung der erhaltenen Hits wurde vor der Sequenzierung das zweite Reportergen lacZ für einen weiteren Test genutzt. Hierzu wurde die Aktivität der ß-Galaktosidase mittels Umsetzung des Substrates FDG durch das Enzym in ein fluoreszentes Produkt bestimmt und so die Hitanzahl vor der Sequenzierung auf 25 % reduziert.

Es ist somit möglich, Adhäsin-Mutanten bereitzustellen, die eine gegenüber dem Wildtyp, insbesondere aber auch gegenüber dem Template verbesserte Äffinität zu CEA und/oder NCA aufweisen. Die Verbesserung der Affinität - erfasst z.B. als Reportergen-Readout nach dem in der EP1721974 A1 bekannten Screeningverfahren - liegt gegenüber dem Template bei mindestens etwa 5 %, vorzugweise bei mindestens 100 %, insbesondere bevorzugt sogar bei mindestens 300 oder mindestens 500 %. Die Affinität gegenüber dem Wildtyp kann dabei auch einen Wert bis zu dem Doppelten oder auch bis zu dem Vierfachen der für das Template angegebenen Werte erreichen.

Besonders hohe Affinitätsverbesserungen werden durch die Kombination von mindestens zwei Mutationen, bevorzugt auch bis zu 3, 5 oder 7 Mutationen erreicht. Eine besonders große Affinitätssteigerung ist vor allem durch eine (ggf. auch mehrfache) Wiederholung des Screeningschrittes nach EP1721974 A1 möglich.

Beabsichtigt ist die Detektion verbesserter Mutanten unterschiedlicher Affinität. Dabei ist es wichtig, den Wildtyp zu finden, um demgegenüber verbesserte Adhäsine zu identifizieren. Damit wird der vollständige Sequenzraum mit in der Affinität verbesserten Mutanten abgedeckt.

Aus diesen Untersuchungen lassen sich die erfindungsgemäßen modifizierten Adhäsine ableiten, die nämlich in einer oder mehreren Aminosäurepositionen verändert sind.

**1. Vorteilhafte Modifikationen**

Ausführungsbeispiel 1 - Position 88 - Threonin

Aus der Literatur (Korotkova et al., The Journal of Biological Chemistry (2006) 281 (39), pp. 29120-29139, "A Subfamily of Dr Adhesins of Escherichia coli Bind Independently to Decay-accelerating Factor and the N-domain of Carcinoembryonic Antigen") ist bekannt, dass die Mutation T88M bei Adhäsinen, vor allem bei DraE zu einer Steigerung der Affinität des Adhäsins gegenüber CEA führt. Daher wurde für die erste Mutagenese die Position T88 gewählt.

Diese sowie alle nachfolgenden Angaben beziehen sich auf die Aminosäurepositionen in DraE nach der Sequenz in Abb. 1.

Die Durchführung des quantitativen Screenings erfolgt wie in der EP1721974 A1 beschrieben.

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:

$$M > L > WT$$

Für die folgenden Mutagenesen wurde die am stärksten verbesserte Mutante an dieser Position, T88M, als Template eingesetzt.

Zusätzlich wurde die Mutation N77K eingeführt, um eine Bindung des Adhäsins an DAF zu verhindern (van Loy et al., Molecular Microbiology (2002) 45(2), pp. 439-452, "Identification of amino acids in Dr adhesin required for binding to decay-accelerating factor"). Diese Mutation hat keine Auswirkung auf die Bindung des Adhäsins an N-CEA (Korotkova et al., The Journal of Biological Chemistry (2006) 281 (39), pp. 29120-29139, "A Subfamily of Dr Adhesins of Escherichia coli Bind Independently to Decay-accelerating Factor and the N-domain of Carcinoembryonic Antigen" und eigene Daten). Damit ergibt sich als Template der nachfolgenden Mutagenesen DraE T88M N77K.

Ausführungsbeispiel 2 - Position 7 - Threonin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:

N>(F,C)>S>V>R,A>(I,L,Y)>WT

Ausführungsbeispiel 3 - Position 17 - Glutamat

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
S>P>K>G,D,R,N>H,Q>WT

Ausführungsbeispiel 4 - Position 22 - Arginin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
T>A>S>N>K>WT

Ausführungsbeispiel 5 - Position 25 - Aspartat

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
S>G>N>A,T>K,R,H,Q,M>WT

Ausführungsbeispiel 6 - Position 27 - Threonin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
K>R>L>V,Y>P,N,Q>WT

Ausführungssbeispiel 7 - Position 28 - Valin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:

$$W = F > WT$$

Ausführungsbeispiel 8 - Position 29 - Alanin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
K > R > S > E, Q > F, G > L, H > P, N, T, W > WT

Ausführungsbeispiel 9 - Position 31 - Threonin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
G = D > S > N > WT

Ausführungsbeispiel 10 - Position 34 - Glutamin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
D >> G > S = N > L = V = T = A > WT

Ausführungsbeispiel 11 - Position 37 - Aspartat

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
N >> WT

Ausführungsbeispiel 12 - Position 38 - Alanin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
S = T > L > WT

Ausführungsbeispiel 13 - Position 39 - Alanin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
Q > S > D, M > G, F > WT

Ausführungsbeispiel 14 - Position 41 - Isoleucin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
V >> WT

Die 141 L-Klone wiesen im Rescreen mittels FDG-Test einen höheren Readout als die I41V-Klone auf. Eine Analyse der Sequenz ergab, dass alle diese Klone noch die zusätzliche Mutation V116A besaßen. Damit stellt auch I41L V116A eine verbesserte Mutante dar.

Ausführungsbeispiel 15 - Position 47 -Glutamin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
T >> N = C > S > G = A = P > WT

Ausführungsbeispiel 16 - Position 52 - Aspartat

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
G > N, S > C, P, Q, Y, H, K, R, T > WT

Ausführungsbeispiel 17 - Position 84 -Asparagin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
D >> S > H > WT

In dem Screening wurde auch die (Kombinations)Mutante Q34L N84S gefunden, die einen höheren Readout im FDG-Rescreen im Vergleich zu N84S aufweist.

Dies zeigt, dass die Kombination von Mutanten zu einer höheren Affinität zu CEA im Vergleich zu der einzelnen Mutante führt.

Ausführungsbeispiel 18 - Position 86 -Arginin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
V >> WT

Ausführungsbeispiel 19 - Position 95 - Threonin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
L >> M, Y, F > (C, W, Q) > N, E, S, I, H > WT
Es wurde auch die kombinierte Mutante T95E T123I gefunden; diese Kombination wies einen höheren FDG-Readout als die Mutante T95E auf.
Dies zeigt, dass die Kombination von Mutanten zu einer höheren Affinität zu CEA im Vergleich zu der einzelnen Mutante führt.

Ausführungsbeispiel 20 - Position 100 - Phenylalanin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
Y > V > WT
Es wurde auch die kombinierte Mutante F100S T11I D37G gefunden; diese Kombination wies einen höheren FDG-Readout als die Mutante F100S auf. Ebenfalls wurde die Kombinationsmutante und F100I T123I gefunden, die einen höheren FDG-Readout als die Mutante F100I zeigte.
Dies zeigt, dass die Kombination von Mutanten zu einer höheren Affinität zu CEA im Vergleich zu der einzelnen Mutante führt.

Ausführungsbeispiel 21 - Position 105 - Valin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
S > A, T > R > M, V, P, N, E > Q, G, K, H > WT

Ausführungsbeispiel 22 - Position 111 - Isoleucin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
C > V > H > Y > T > M = F > WT

Ausführungsbeispiel 23 - Position 114 - Isoleucin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
V >> L > A = C > WT

Ausführungsbeispiel 24 - Position 115 - Tyrosin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
T = W > E > V > WT

Ausführungsbeispiel 25 - Position 116 - Valin

Die Durchführung des quantitativen Screenings erfolgt wie in der EP1721974 A1 beschrieben; zusätzlich wurde ein Rescreen durchgeführt (s.o.)

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
A > S > L > WT

Ausführungsbeispiel 26 - Position 118 - Glycin

Die Durchführung des quantitativen Screenings erfolgt wie in der EP1721974 A1 beschrieben; zusätzlich wurde ein Rescreen durchgeführt (s.o.).

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
P > S > WT

Es wurde auch die kombinierte Mutante G118S 185L gefunden; diese Kombination wies einen höheren FDG-Readout im Vergleich zu G118S auf und war unter den ersten Hits zu finden.

Dies zeigt, dass die Kombination von Mutanten zu einer höheren Affinität zu CEA im Vergleich zu der einzelnen Mutante führt.

Zusammenfassung der Ausführungsbeispiele 1 bis 26

Basierend auf folgender Sequenz zu DraE N77K werden alle folgenden Konsensussequenzen angegeben (s. auch Abb. 1):
GFTPSGTTGTTKLTVTEECQVRVGDLTVAKTRGQLT-DAAPIGPVTVQALGCDARQVALKAD TDNFE-QGKFFLISDNKRDKLYVNIRPTDNSAWTTDNGVFY-KNDVGSWGGIIGIYVDGQQTNT PPGNYTLTLTGGY-WAK

Aus diesen Ausführungsbeispielen ergibt sich folgende "positive" Konsensussequenz für die Aminosäuren, deren Mutation innerhalb des Adhäsin-Gens zu einer Verbesserung der Affinität des Adhäsins an CEA führt:

Konsensussequenz des Alignments in Abb. 12

XXXXXX T7(N, F, C, S, V, R, A, I, L, Y) XXXXXXXXX E17 (S, P, K, G, D, R, N, H, Q) XXXX R22 (T, A, S, N, K) XX D25(S, G, N, A, T, K, R, H, Q, M) X T27(K, R, L, V, Y, P, N, Q) V28(W, F) A29(K, R, S, E, Q, F, G, L, H, P, N, T, W) X T31(G, D, S, N) XX Q34(D, G, S, N, L, V, T, A) XX D37N A38(S, T, L) X A39(Q, S, D, M, G, F) X 141 (V) XXXXX Q47(T, N, C, S, G, A, P) XXXX D52(G, N, S, C, P, Q, Y, H, K, R, T) XXXXXXXXXXXXXXXXXXXXXXXXXXXX N84(D, S, H) X R86V X T88(M, L) XXXXXX T95(L, M, Y, F, C, W, Q, N, E, S, 1, H) XXXX F100(Y, V) XXXX V105(S, A, T, R, M, V, P, N, E, Q, G, K, H) XXXXX I111(C, V, H, Y, T, M, F) XX I114(V, L, A, C) Y115(T, W, E, V) V116(A, S, L) X G118(P, S) XXXXXXXXXXXXXXXXXXXX

Das Alignment ist in Abb. 12 dargestellt.

In einer vorteilhaften Ausführungsform weisen die erfindungsgemäßen modifizierten Adhäsine eine oder mehrere der folgenden Aminosäuren auf: L26, G42, P43, V44, L49, D89, S91, S107, W108, G110. In einer besonders bevorzugten Ausführungsform sind alle diese Aminosäuren vorhanden.

Daraus ergibt sich vorteilhafterweise ein Konsensus wie folgt:

Konsensussequenz des Alignments in Abb. 13

XXXXXX T7(N, F, C, S, V, R, A, I, L, Y) XXXXXXXXX E17 (S, P, K, G, D, R, N, H, Q) XXXX$_4$ R22 (T, A, S, N, K) XX D25(S, G, N, A, T, K, R, H, Q, M) L26 T27(K, R, L, V, Y, P, N, Q) V28(W, F) A29(K, R, S, E, Q, F, G, L, H, P, N, T, W) X T31(G, D, S, N) XX Q34(D, G, S, N, L, V, T, A) XX D37N A38(S, T, L) X A39(Q, S, D, M, G, F) X I41(V) G42 P43 V44 XX Q47(T, N, C, S, G, A, P) X L49 XX D52(G, N, S, C, P, Q, Y, H, K, R, T) XXXXXXXXXXXXXXXXXXXXXXXXXXXXX N84(D, S, H) X R86V X T88(M, L) D89 X S91 XXX T95(L, M, Y, F, C, W, Q, N, E, S, I, H) XXXX F100(Y, V) XXXX V105 (S, A, T, R, M, V, P, N, E, Q, G, K, H) X S107 W108 X G110 I111(C, V, H, Y, T, M, F) XX I114(V, L, A, C) Y115 (T, W, E, V) V116(A, S, L) X G118(P, S) XXXXXXXXXXXXXXXXXXXX

Das Alignment ist in Abb. 13 dargestellt. Dabei sind die vorteilhaft vorhandenen Aminosäuren unterstrichen.

Es ist von Vorteil, wenn die erfindungsgemäßen Adhäsine außerdem eine oder mehrere, insbesondere alle, der folgenden Aminosäuren aufweisen: C19, C51, G106, Y128, T129.

Demnach weisen die erfindungsgemäßen Adhäsine vorteilhafterweise einen Konsensus wie folgt auf:
Konsensussequenz aus Abb. 14, dabei sind alle vorteilhaft vorhandenen Aminosäuren unterstrichen; Kombination aus Konsensusstrukturen aus Abb. 12 und 13 sowie den weiteren obigen Angaben zu bevorzugten Aminosäuren):

Konsensussequenz aus Abbildung 14:

XXXXXX T7(N, F, C, S, V, R, A, I, L, Y) XXXXXXXXX E17 (S, P, K, G, D, R, N, H, Q) X C19 XX R22(T, A, S, N, K) XX D25(S, G, N, A, T, K, R, H, Q, M) L26 T27(K, R, L, V, Y, P, N, Q) V28(W, F) A29(K, R, S, E, Q, F, G, L, H, P, N, T, W) X T31 (G, D, S, N) XX Q34 (D, G, S, N, L, V, T, A) XX D37N A38(S, T, L) A39(Q, S, D, M, G, F) X 141 (V) G42 P43 V44 XX Q47(T, N, C, S, G, A, P) X L49 X C51 D52(G, N, S, C, P, Q, Y, H, K, R, T) XXXXXXXXXXXXXXXXXXXXXXXXXXXXX N84 (D, S, H) X R86V X T88(M, L) D89 X S91 XXX T95(L, M, Y, F, C, W, Q, N, E, S, I, H) XXXX F100(Y, V) XXXX V105(S, A, T, R, M, V, P, N, E, Q, G, K, H) G106 S107 W108 X G110 I111(C, V, H, Y, T, M, F) XX I114(V, L, A, C) Y115(T, W, E, V) V116(A, S, L) X G118(P, S) XXXXXXXXX Y128 T129 XXXXXXXXXX

## 2. Besonders bevorzugte Kombinationen von Mutationen

Die folgende Kombination von Einfachmutanten hat sich als besonders vorteilhaft erwiesen:

Eine Mutagenese auf dem Wildtyp-Adhäsin DraE hat zu der verbesserten Mutante T88M geführt (s. Ausführungsbeispiel 1) und bei weiteren Mutageneserunden mit dieser Mutante DraE T88M (N77K) als Template wurden weitere verbesserte Mutanten detektiert (s. Ausführungsbeispiele 2 bis 26). So wurde z. B. die Mehrfachmutante DraE T88M (N77K) I111H generiert, die eine deutlich erhöhte Affinität zu CEA gegenüber dem Wildtyp-Adhäsin DraE und der Mutante DraE T88M zeigt.

Ein weiteres Beispiel ist das Konstrukt DraE T88M Q34L N84S, das eine höhere Affinität zu CEA aufweist als DraE T88M N84S (s. Ausführungsbeispiel 17). Auch die Kombinationsmutante DraE T88M G118S I85L hat im Vergleich zu der Mutante DraE T88M G118S eine höhere Affinität zu CEA (Ausführungsbeispiel 26).

Eine im Vergleich zu F110S erhöhte Affinität gegenüber CEA wies die Kombinationsmutante F100S T11I D37G auf (s. Ausführungsbeispiel 20). Dies war auch der Fall bei der Kombinationsmutante F100I T123I, die eine erhöhte Affinität gegenüber CEA im Vergleich zu F100I aufwies (s. Ausführungsbeispiel 20). Es wurde auch die kombinierte Mutante T95E T123I gefunden; diese Kombination wies eine höhere Affinität zu CEA auf als die Mutante T95E (s. Ausführungsbeispiel 19).

Damit werden zur Lösung der Aufgabe der Erfindung bevorzugte Ausführungsformen eines in der Affinität zu CEA und/oder NCA verbesserten Adhäsin-Liganden definiert, die jeweils irgendeine denkbare Kombinationen der Einfachmutanten, die in der Konsensussequenz 1 aufgeführt sind, aufweisen.

Die aufgeführten Ausführungsbeispiele zeigen, dass mit dem hier verwendeten nHybrid/Two-Hybrid Screeningverfahren durch die Durchführung von repetitiven Zyklen die Generierung von Kombinationsmutanten mit erhöhten Affinitäten zu CEA und/oder NCA möglich ist.

## 3. Mehrfach-Mutagenese

Diese Experimente wurden im nHybrid-System durchgeführt (EP1721974 A1).

Zum Einen wurde DraE T88M N77K I111V (TM = Triplemutante) als Template der Mutagenese eingesetzt, zum Anderen wurde DraE T88M N77K I111V I114V V116A (FM = Fünffachmutante), die eine noch höhere Affinität zu CEA besitzt, verwendet.

Ausführungsbeispiel 27 - Position 39 - Alanin

a) TM als Template

Die Durchführung des quantitativen Screenings erfolgt wie in der EP1721974 A1 beschrieben; zusätzlich wurde ein Rescreen durchgeführt (s.o.).

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
S > G > D = H > Q > WT
Bei DraE T88M N77K als Template war die Reihenfolge der verbesserten Mutanten Q > S > D > M > G > F, das bedeutet, dass die TM voraussichtlich eine veränderte Struktur besitzt, die dazu führt, dass an der Position 39 unterschiedliche Mutanten bzw. die bekannten Mutanten in veränderter Reihenfolge gefunden werden.

b) FM als Template

Die Durchführung des quantitativen Screenings erfolgt wie in der EP1721974 A1 beschrieben; zusätzlich wurde ein Rescreen durchgeführt (s.o.).

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
S > WT
Bei DraE T88M N77K als Template war die Reihenfolge der verbesserten Mutanten Q > S > D > M > G > F, das bedeutet, dass die FM voraussichtlich eine veränderte Struktur besitzt, die dazu führt, dass an der Position 39 unterschiedliche Mutanten bzw. die bekannten Mutanten in veränderter Reihenfolge gefunden werden.

c) Vergleich der beiden Templates

TM: S > G > D = H > Q > WT
FM: S > WT

Ausführungsbeispiel 28 - Position 41 - Isoleucin

a) TM als Template

Die Durchführung des quantitativen Screenings erfolgt wie in der EP1721974 A1 beschrieben; zusätzlich wurde ein Rescreen durchgeführt (s.o.).

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
V > L > T > WT

Das bedeutet, dass eine Mutation an dieser Stelle auch bei der TM als Template noch eine Verbesserung in der Affinität zu CEA bewirkt; dabei tritt in diesem Fall das gleiche Mutantenspektrum auf wie bei DraE T88M N77K als Template. Diese Tatsache lässt darauf schließen, dass die Strukturänderung bei der TM keinen großen Einfluss auf die Aminosäureposition 41 hat.

b) FM als Template

Die Durchführung des quantitativen Screenings erfolgt wie in der EP1721974 A1 beschrieben; zusätzlich wurde ein Rescreen durchgeführt (s.o.).

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
L > WT

Bei DraE T88M N77K als Template war L als schwach verbesserte Mutante detektiert worden; V war die beste Mutante. Das bedeutet, dass die FM voraussichtlich eine veränderte Struktur besitzt, die dazu führt, dass an der Position 41 unterschiedliche Mutanten bzw. die bekannten Mutanten in veränderter Reihenfolge gefunden werden.

c) Vergleich der beiden Templates

TM: V > L > T > WT
FM: L > WT

Ausführungsbeispiel 29 - Position 47 - Glutamin

a) TM als Template

Die Durchführung des quantitativen Screenings erfolgt wie in der EP1721974 A1 beschrieben; zusätzlich wurde ein Rescreen durchgeführt (s.o.).

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
S > A > C > T > WT

Bei DraE T88M N77K als Template war die Reihenfolge der verbesserten Mutanten T > N = C > S > G = A = P > WT, das bedeutet, dass die TM voraussichtlich eine veränderte Struktur besitzt, die dazu führt, dass an der Position 47 unterschiedliche Mutanten bzw. die bekannten Mutanten in veränderter Reihenfolge gefunden werden.

b) FM als Template

Die Durchführung des quantitativen Screenings erfolgt wie in der EP1721974 A1 beschrieben; zusätzlich wurde ein Rescreen durchgeführt (s.o.).

(i) Sequenzierung und Auswertung der Hits

Die Sequenzierung der DNA aus den Hits nach Transformation in Bakterien ergibt folgende Reihenfolge an in der Affinität zu CEA verbesserten Mutanten basierend auf der Häufigkeit der identifizierten Hits:
S > WT

Bei DraE T88M N77K als Template war die Reihenfolge der verbesserten Mutanten T > N = C > S > G = A = P > WT, das bedeutet, dass die FM voraussichtlich eine veränderte Struktur besitzt, die dazu führt, dass an der Position 47 unterschiedliche Mutanten bzw. die bekannten Mutanten in veränderter Reihenfolge gefunden werden.

c) Vergleich der beiden Templates

TM: S > A > C > T > WT
FM: S > WT

Mit diesen Ausführungsbeispielen wurde erneut gezeigt, dass mit dem hier verwendeten nHybrid Screeningverfahren durch die Durchführung von repetitiven Zyklen die Generierung von Kombinationsmutanten mit erhöhten Affinitäten zu CEA möglich ist

**3. Analyse des *in vivo* Abbaus der erfindungsgemäß einsetzbaren Nanopartikel mittels High Content Screening**

Ziel dieser Untersuchung war der Nachweis des biologischen Abbaus des Dipeptidgecoateter Nanopartikels (Core CdSe, Shell ZnS, Emissionsmaximum 655 nm, hydrodynamischer Durchmesser von etwa 12.5 nm ohne Proteinligand) in ausgewählten Organen der Maus durch einen Vergleich der Fluoreszenz am Tag 2 und 42 nach intravenöser Injektion von fluoreszenten Nanopartikelkonjugaten mit einer Dosis 1,7 mg/kg Körpergewicht.

## Methode

6 NMRI Mäusen wurde über die Schwanzvene 1,7 mg/kg Körpergewicht des fluoreszenten Nanopartikelkonjugates (QD655#D416-36-KNH - Proteinligand Klon 5582 (sog. Fünfach-Mutante FM, siehe oben) injiziert. Die Mäuse wurden am Tag 2 bzw. 42 nach Injektion jeweils getötet, die Organe wurden entnommen und bei -80°C schockgefroren.

Probenherstellung:

200 mg des jeweiligen Organs wurden eingewogen und mit 1 ml 500 mM Bicine Puffer (pH = 8.3) in Cryoröhrchen und 1g Homogenisierungs-Beads (MatrixD von MP Biomedicals) 1 min homogenisiert mit dem Homogenisator Fastprep24 (MP Biomedicals). Das Homogenat wurde auf Eis gestellt.

Ansatz Proben-Einbettung (alle Angaben in $\mu$l)

| BSA-Lösung 30% | 106,4 |
|---|---|
| Homogenat | 213,6 |
| Glutaraldehyd-Lösung 25% | 17,0 |

Die BSA-Lösung wurde in einem Eppendorf-Gefäß vorgelegt, dann das Homogenat zugegeben und die Probe für 2 min auf Eis gekühlt. Dann wurde die Glutaraldehyd-Lösung zugegeben und schnell gemischt. 200 $\mu$l der Proben wurden in jeweils ein well einer Micro-Titer-Platte mit Deckglas-Boden (Greiner) pipettiert. Die Fixierung durch Glutaraldehyd dauerte wenige Minuten und führte zu einem festen Gel.

Messung der Rohdaten

Die Mikroskopie erfolgte mit Olympus Scan^R System mit einem 60x/1.2 W Objektiv. Bilder der Proben wurden generiert über die Scan^R Aquisition Software: 16 Stapel pro Probe (= pro well) wurden aufgenommen mit jeweils 40 frames/Stapel mit 0,5 $\mu$m Abstand zwischen den frames. Es wurden Aufnahmen in 2 Kanälen parallel gemacht: Die Belichtungszeit im Nanopartikel-Kanal (Emissionsfilter 655/16) lag bei 200 ms, im Autofluoreszenzkanal (Emissionsfilter 525/30) bei 1000ms.

Angaben zur Aufnahmefläche bzw. Volumen:

| Breite | 109 $\mu$m |
|---|---|

(fortgesetzt)

| Länge | 109 $\mu$m |
|---|---|
| Höhe | 20 $\mu$m |
| Volumen/Stack | 2,38E-10 l |
| Volumen gesamt | 3,80E-09 l |

Auswertung der Rohdaten

Die Quantifizierung der Nanopartikel erfolgte mittels Scan^R Analysis Software. Zuerst wurde eine Maximalprojektion der Stapel entlang der z-Achse auf eine Ebene generiert, dann wurde eine Glättung mittels Rolling Ball durchgeführt und zuletzt wurde der gewichtete Autofluoreszenzkanal vom Nanopartikel-Kanal abgezogen. Der Intensity threshold zur Segmentierung wurde auf 43 gesetzt.

Die gefundenen Nanopartikel wurden mittels Gating in 3 Kategorien eingeteilt (small, middle und big), das Kriterium war die Fläche der gefundenen Nanopartikel, innerhalb eines sinnvollen Korridors, der durch die Mean Intensity definiert wurde (Grauwertstufen im Bereich 60 - 400). Die Gates waren rechteckig und wurden wie folgt definiert:

small: 4 - 35 Pixel
middle: 36 - 400 Pixel
big: 401 - 100 000 Pixel

Die meisten Objekte wurden im Gate small gefunden, die wenigsten in big.

Aufgrund des Auftretens großer und mittelgroßer Aggregate ist eine genaue quantitative Aussage nicht möglich, da nicht exakt zu ermitteln ist, wie viele einzelne Nanopartikel jeweils ein Aggregat bilden. Allerdings kann über die Fluoreszenz als dimensionslose Grösse, die annähernd proportional zur Anzahl der Nanopartikel in einem Aggregat ist, eine qualitative Aussage über die Nanopartikel-Verteilung und die Pharmakokinetik gemacht werden.

*Berechnung der dimensionslosen Fluoreszenz:*

Die Objektzahl wird mit der Mean Total Intensity (mittlere Gesamt-Fluoreszenzintensität pro Objekt) der jeweiligen Nanopartikel-Klasse multipliziert:

$$\text{Objects} \times \text{Mean Intensity} = \text{Fluoreszenz}$$

Die Fluoreszenz der Homogenate aus Lebern, Milzen und Lungen an den Tagen 2 und 42 wurden berechnet und davon der Mittelwert bestimmt. Die Fluoreszenz an Tag 2 wurde jeweils auf 100 % normiert.

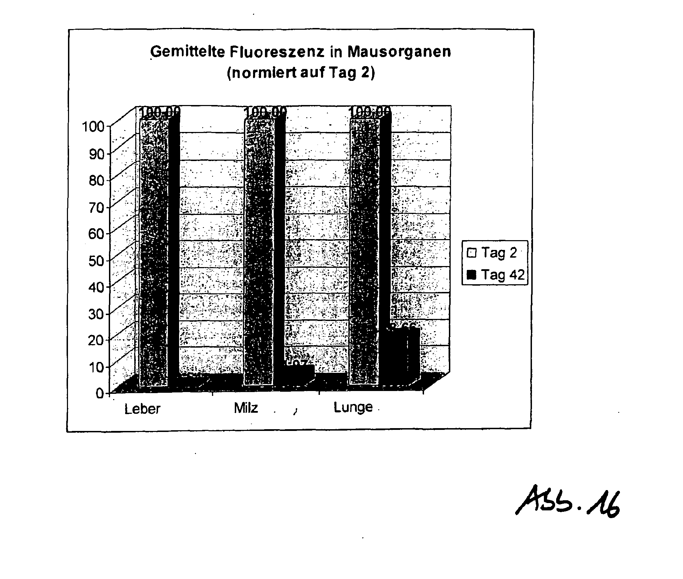

Die Ergebnisse sind in Abb. 16 zusammengefasst. Danach waren am Tag 42 im Vergleich zum Tag 2 in der Leber nur noch 0,54, in der Milz nur noch 4,97 und in der Lunge nur noch 18,66 % der Fluoreszenz detektierbar. Das zeigt, dass die erfindungsgemäßen Adhäsingekoppelten Nanopartikel *in vivo* abgebaut werden.

**Patentansprüche**

1. Adhäsin umfassend eine Aminosäuresequenz, die ausgewählt ist von der Gruppe bestehend aus folgenden Sequenzen

   a. DraE Gruppe wie dargestellt in Abbildung 5
   b. DraE/AfaE-III wie dargestellt in Abbildung 6
   c. DraE wie dargestellt in Abbildung 1

   wobei das Adhäsin eine oder mehrere der folgenden Mutationen aufweist, und N-terminal um bis zu 18, bevorzugt bis zu 12, 10 oder 5 und/oder C-terminal um bis zu 10, bevorzugt um bis zu 8 oder 5 Aminosäuren deletiert sein kann:

   T7(N, F, C, S, V, R, A, I, L, Y); E17 (S, P, K, G, D, R, N, H, Q); R22(T, A, S, N, K); D25(S, G, N, A, T, K, R, H, Q, M); T27(K, R, L, V, Y, P, N, Q); V28(W, F); A29(K, R, S, E, Q, F, G, L, H, P, N, T, W); T31 (G, D, S, N); Q34(D, G, S, N, L, V, T, A); D37N; A38(S, T, L); A39(Q, S, D, M, G, F); I41 (V); Q47(T, N, C, S, G, A, P); D52(G, N, S, C, P, Q, Y, H, K, R, T); N84(D, S, H); R86V; T88(M, L); T95(L, M, Y, F, C, W, Q, N, E, S, I, H); F100(Y, V); V105(S, A, T, R, M, V, P, N, E, Q, G, K, H); I111(C, V, H, Y, T, M, F) 1114(V, L, A, C); Y115(T, W, E, V); V116(A, S, L); G118(P, S),

   wobei das Adhäsin bei Vorhandensein der Mutationen T88M und/oder N77K wenigstens eine weitere der oben genannten Mutationen aufweist.

2. Adhäsin nach Anspruch 1, **dadurch gekennzeichnet, dass** es an die N-terminale Sequenz eines Bindungsproteins mit einer Aminosäuresequenz binden kann, die ausgewählt ist aus der Gruppe der folgenden Sequenzen

   a. CEA/NCA (Sequenz aus Abb. 9)
   b. CEA (Sequenz aus Abb. 10) und/oder NCA (Sequenz aus Abb. 11).

3. Adhäsin nach einem der Ansprüche 1 oder 2, **dadurch gekennzeichnet, dass** das Adhäsin gegenüber dem Bindungsprotein nach Anspruch 2, insbesondere zu der N-terminalen Sequenz von CEA, eine um mindestens 5%, vorzugsweise mindestens 100%, insbesondere mindestens 300% oder 500% verbesserte Affinität gegenüber dem Wildtyp aufweist.

4. Adhäsin nach einem der vorherigen Ansprüche, **dadurch gekennzeichnet, dass** es eine oder mehrere der folgenden Mutationen aufweist:

   V28W; V28F; A39Q; A39S; I41L; Q47S; Q47T; I85L; T95L; G118S und T123I.

5. Adhäsin mit einer Sequenz nach Abb. 15.

6. Nanopartikel konjugiert mit einem Adhäsin nach einem oder mehreren der obigen Ansprüche.

7. Nanopartikel nach Anspruch 6, **dadurch gekennzeichnet, dass** er einen anorganischen Kern und eine Passivierungsschicht aufweist und der kleinste Durchmesser des anorganischen Kerns mit der Passivierungsschicht nicht mehr als 15 nm, beträgt, vorzugsweise nicht mehr als 10 nm, besonders bevorzugt nicht mehr als 5 nm.

8. Nanopartikel nach einem der Ansprüche 6 oder 7, **dadurch gekennzeichnet, dass** der Nanopartikel nicht-inert ist.

9. Verwendung des Adhäsins nach einem der Ansprüche 1 bis 5 oder des Nanopartikels nach einem der Ansprüche 6 bis 8 zur Herstellung eines Kontrastmittels für die medizinische Anwendung.

10. Verwendung des Adhäsins nach einem der Ansprüche 1 bis 5 oder des Nanopartikels nach einem der Ansprüche 6 bis 8 zur Herstellung eines Kontrastmittels für die Identifizierung von Darmkrebs, insbesondere von CRC-Zellen.

**Claims**

1. Adhesin comprising an amino acid sequence which is selected from the group consisting of the following sequences

   a. DraE group as shown in Figure 5
   b. DraE/AfaE-III as shown in Figure 6
   c. DraE as shown in Figure 1

   **wherein** the adhesin has one or more of the following mutations and may be deleted N-terminally by up to 18, preferably by up to 12, 10 or 5, and/or C-terminally by up to 10, preferably by up to 8 or 5 amino acids:

   T7(N, F, C, S, V, R, A, I, L, Y); E17 (S, P, K, G, D, R, N, H, Q); R22(T, A, S, N, K); D25(S, G, N, A, T, K, R, H, Q, M); T27(K, R, L, V, Y, P, N, Q); V28(W, F); A29(K, R, S, E, Q, F, G, L, H, P, N, T, W); T31(G, D, S, N); Q34(D, G, S, N, L, V, T, A); D37N; A38(S, T, L); A39(Q, S, D, M, G, F);

I41(V); Q47(T, N, C, S, G, A, P); D52(G, N, S, C, P, Q, Y, H, K, R, T); N84(D, S, H); R86V; T88 (M, L); T95(L, M, Y, F, C, W, Q, N, E, S, I, H); F100(Y, V); V105(S, A, T, R, M, V, P, N, E, Q, G, K, H); I111(C, V, H, Y, T, M, F) I114(V, L, A, C); Y115(T, W, E, V); V116(A, S, L); G118(P, S),

**wherein** the adhesin has at least one further of the abovementioned mutations if the mutations T88M and/or N77K are present.

2. Adhesin according to Claim 1, **characterized in that** it is capable of binding to the N-terminal sequence of a binding protein having an amino acid sequence which is selected from the group of the following sequences

   a. CEA/NCA (sequence of Fig. 9)
   b. CEA (sequence of Fig. 10) and/or NCA (sequence of Fig. 11).

3. Adhesin according to either of Claims 1 and 2, **characterized in that** the adhesin has an affinity for the binding protein according to Claim 2, in particular for the N-terminal sequence of CEA, which is improved by at least 5%, preferably by at least 100%, in particular at least 300% or 500% over that of the wild type.

4. Adhesin according to any of the preceding claims, **characterized in that** it has one or more of the following mutations:

   V28W; V28F; A39Q; A39S; I41L; Q47S; Q47T; I85L; G118S and T123I.

5. Adhesin having a sequence according to Fig. 15.

6. Nanoparticle, conjugated to an adhesin according to one or more of the above claims.

7. Nanoparticle according to Claim 6, **characterized in that** it has an inorganic core and a passivation layer, with the smallest diameter of the inorganic core together with the passivation layer being no more than 15 nm, preferably no more than 10 nm, especially preferably no more than 5 nm.

8. Nanoparticle according to either of Claims 6 and 7, **characterized in that** the nanoparticle is non-inert.

9. The use of the adhesin according to any of Claims 1 to 5 or of the nanoparticle according to any of Claims 6 to 8 for the preparation of a contrast agent for medical use.

10. The use of the adhesin according to any of Claims 1 to 5 or of the nanoparticle according to any of Claims 6 to 8 for the preparation of a contrast agent for identifying bowel cancer, in particular CRC cells.

**Revendications**

1. Adhésine comprenant une séquence d'acides aminés qui est sélectionnée dans le groupe composé des séquences suivantes :

   a. groupe DraE tel que représenté dans le schéma 5,
   b. DraE/AfaE-III, tel que représenté dans le schéma 6,
   c. DraE, tel que représenté dans le schéma 1,

   où l'adhésine présente une ou plusieurs des mutations suivantes, et où la partie N-terminale peut présenter une délétion de jusqu'à 18, de préférence jusqu'à 12, 10 ou 5, et/ou la partie C-terminale jusqu'à 10, de préférence jusqu'à 8 ou 5, acides aminés :

   T7(N, F, C, S, V, R, A, I, L, Y) ; E17 (S, P, K, G, D, R, N, H, Q) ; R22(T, A, S, N, K) ; D25(S, G, N, A, T, K, R, H, Q, M) ; T27(K, R, L, V, Y, P, N, Q) ; V28(W, F) ; A29(K, R, S, E, Q, F, G, L, H, P, N, T, W) ; T31 (G, D, S, N) ; Q34(D, G, S, N, L, V, T, A) ; D37N ; A38(S, T, L) ; A39(Q, S, D, M, G, F) ; 141 (V) ; Q47(T, N, C, S, G, A, P) ; D52(G, N, S, C, P, Q, Y, H, K, R, T) ; N84(D, S, H) ; R86V ; T88(M, L) ; T95(L, M, Y, F, C, W, Q, N, E, S, I, H) ; F100(Y, V) ; V105(S, A, T, R, M, V, P, N, E, Q, G, K, H) ; I111(C, V, H, Y, T, M, F) ; I114(V, L, A, C) ; Y115(T, W, E, V) ; V116 (A, S, L) ; G118(P, S),

   où l'adhésine, en cas de présence des mutations T88M et/ou N77K, présente au moins une autre des mutations précédemment citées.

2. Adhésine selon la revendication 1, **caractérisée en ce qu'**elle peut se lier à la séquence N-terminale d'une protéine de liaison ayant une séquence d'acides aminés qui est sélectionnée dans le groupe des séquences suivantes :

   a. CEA/NCA (séquence représentée dans le schéma 9),
   b. CEA (séquence représentée dans le schéma 10) et/ou NCA (séquence représentée dans le schéma 11).

3. Adhésine selon l'une des revendications 1 ou 2, **caractérisée en ce que** l'adhésine présente eu égard à la protéine de liaison selon la revendication 2, en particulier à la séquence N-terminale de CEA, une affinité améliorée d'au moins 5 %, de préférence d'au moins 100 %, en particulier d'au moins 300 % ou 500 %, par rapport au type sauvage.

4. Adhésine selon l'une des revendications précédentes, **caractérisée en ce qu'**elle présente une ou plusieurs des mutations suivantes :

V28W ; V28F ; A39Q ; A39S ; I41L ; Q47S ; Q47T ; I85L ; T95L ; G118S et T123I.

5. Adhésine présentant une séquence selon le schéma 15.

6. Nanoparticule conjuguée avec une adhésine selon une ou plusieurs des revendications précédentes.

7. Nanoparticule selon la revendication 6, **caractérisée en ce qu'**elle présente un noyau inorganique et une couche de passivation et **en ce que** le plus petit diamètre du noyau inorganique avec la couche de passivation n'est pas supérieur à 15 nm, de préférence pas supérieur à 10 nm, de façon particulièrement préférée pas supérieur à 5 nm.

8. Nanoparticule selon l'une des revendications 6 ou 7, **caractérisée en ce que** la nanoparticule est non-inerte.

9. Utilisation de l'adhésine selon l'une des revendications 1 à 5 ou de la nanoparticule selon l'une des revendications 6 à 8 pour fabriquer un agent de contraste destinée à une application en médecine.

10. Utilisation de l'adhésine selon l'une des revendications 1 à 5 ou de la nanoparticule selon les revendications 6 à 8 pour fabriquer un agent de contraste destiné à l'identification du cancer du côlon, en particulier des cellules de cancer colorectal (CRC).

Abb. 1 ; SEQ ID No 1

EP 2 250 190 B1

```
GFTPSGTTGT TKLTVTEECQ VRVGDLTVAK TRGQLTDAAP IGPVTVQALG 50
CDARQVALKA DTDNFEQGKF FLISDNKRDK LYVNIRPTDN SAWTTDNGVF 100
YKNDVGSWGG IIGIYVDGQQ TNTPPGNYTL TLTGGYWAK        139
```

EP 2 250 190 B1

CLUSTAL 2.0.8 multiple sequence alignment jeweils einem
Vertreter jeder Adhäsin-Gruppe (laut Korotkova, 2007)

*Abb. 2*

```
AfaE-5     AFTGSGSTGTTKLTVTEQCQVLVTGSD--VTKTRGELTDGARVGVLSVTAKGCNT-EHAA 57
DrbE-122   AFTASGNTGTTKLTVTEQCQVMVTGSA--STKTRGELIDGARVGALSLNARGCNT-EHAA 57
DraE       GFTPSGTTGTTKLTVTEECQVRVGDLT--VAKTRGQLTDAAPIGPVTVQALGCDA-RQVA 57
SM254      QFSYSGNTGTVKVTVTEECQIQVGDFS--TTKPRSQLTNGAAIGPINVTARGCDT-RQIA 57
DaaE       TFQASGTTGITTLTVTEECRVQVGNVT--ATLARSKLKDDTAIGVIGVTALGCNG-LQAA 57
AfaE-2     -AVDKHATGTYTLNVTEECAVHLTDNT--ESLRKTDLTEGKLLAGVGLSATGCAN-SKVA 56
AfaE-1     NFTSSGTNGKVDLTITEECRVTVESKS--ESFLRSGLVANRHITNLGIQSTGCGTGQRVA 58
NfaE-111   GIRLGTATASGTITNMESCTVNLTIATPDAKMNRAGMQENREITKFKVASNDCPTDTYAV 60
                            : *.*  : :            : :      :  . : : .*       .

AfaE-5     LRAQPDNYHQGK-IVLIRDDYQARINVRLQAT-----DGRAWNTNGDTVYRADAGNWGGS 111
DrbE-122   LRAQADNYHNGK-IVLLREDQQARINVRLVAS-----DGGQWTNDGATTYRDAAGDWGGS 111
DraE       LKADTDNFEQGK-FFLISDNNRDKLYVNIRPT-----DNSAWTTDNGVFYKNDVGSWGGI 111
SM254      LQAGADNIEGDK-LYMRSDNGGDKLYVVLSAL-----DGSNWTTDNGVFYNTVPGNWGGT 111
DaaE       LQADPDNYDATN-LYMTSRN-HDKLNVKLKAT-----DGSSWTYGNGVFYKTEGGNWGGH 110
AfaE-2     FSADAGNLKGTN-ILLKGADKTSFIPVYWETATAAGDDSHNWTATTEGIHRNSNGPWEGT 115
AfaE-1     LKLGAGSYDDTNGAHMTHENGTDKLLVSMGSA------TGDGTQDGGVYYINRDGNWNGQ 112
NfaE-111   WFKEIDNVANGI--AQGKSEYQTRFYLRMAST-----NGTESQKDISVGNKTGKGLSGKL 113
                   ..     :    : :  .                            *

AfaE-5     LF------VVVDGDNVDKPTGSYTLNLDWGYWVS 139
DrbE-122   LY------VVVDGDNTSNQAGSYTLNMDGGYWAS 139
DraE       IG------IYVDGQQTNTPPGNYTLTLTGGYWAK 139
SM254      IG------VKVQGDQTRTPTGNYTLTLTGGYWAK 139
DaaE       VG------ISVDGNQTDKPTGEYTLNLTGGYWTN 138
AfaE-2     IR------LRVIGDQTSAKAQAYTLVLNGGTWIE 143
AfaE-1     MV------FIVRNDQQHLPTGKYTLNLEGGFWTK 140
NfaE-111   ANGAFEGKITLAQDTTGVPVDVYTYNLMAAVYSQ 147
                    . :  :           ** :  . : .
```

ohne Signalsequenz

Abb.3, SEQ ID No2

```
XXXXXXXXXXXXXXXXXEXCXXXXXXXX--XXXXXXXXXXXXXXXXXXXXXXXXCXX-
XXXXXXXXXXXXXXXX-XXXXXXXXXXXXXXXXXXX-----
XXXXXXXXXXXXXXXXXGXXXXXXX------XXXXXXXXXXXXXXXYTXXXXXXXXX
```

CLUSTAL 2.0.8 multiple sequence alignment von Adhäsinen der DraE-group (laut Korotkova, 2007)

```
G2152     GFTPSGTTGTTKLTVTEECQVRVGDLTVAKTRGQLTDAAPIGPVTVQALGCDARQVALKA 60        G2152     TNTPPGNYTLALTGGYWAK 139
SM297     GFTPSGTTGTTKLTVTEECQVRVGDLTVAKTRGQLTDAAPIGPVTVQALGCDARQVALKA 60        SM297     TNTPPGNYTLTLTGGYWAK 139
JJB30     GFTPSGTTGTTKLTVTEECQVRVGDLTVAKTRGQLTDAAPIGPVTVQALGCDARQVALKA 60        JJB30     TNTPPGNYTLNLTGGYWAK 139
SM437     GFTPSGTTGTTKLTVTEECQVRVGDLTVAKTRGQLTDAAPIGPVTVQALGCDARQVALKA 60        SM437     TNTPPGNYTLTLTGGYWAK 139
SM249     GFTPSGTTGTTKLTVTEECQVRVGDLTVAKTRGQLTDAAPIGPVTVQALGCDARQVALKA 60        SM249     TNTPPGNYTLTLTGGYWAK 139
SM246     GFTPSGTTGTTKLTVTEECQVRVGDLTVAKTRGQLTDAAPIGPVTVQALGCDARQVALKA 60        SM246     TNTPPGNYTLTLTGGYWAK 139
SM245     GFTPSGTTGTTKLTVTEECQVRVGDLTVAKTRGQLTDAAPIGPVTVQALGCDARQVALKA 60        SM245     TNTPPGNYTLTLTGGYWAK 139
SM54      GFTPSGTTGTTKLTVTEECQVRVGDLTVAKTRGQLTDAAPIGPVTVQALGCDARQVALKA 60        SM54      TNTPPGNYTLTLTGGYWAK 139
G2171     GFTPSGTTGTTKLTVTEECQVRVGDLTVAKTRGQLTDAAPIGPVTVQALGCDARQVALKA 60        G2171     TNTPPGNYTLTLTGGYWAK 139
G2166     GFTPSGTTGTTKLTVTEECQVRVGDLTVAKTRGQLTDAAPIGPVTVQALGCDARQVALKA 60        G2166     TNTPPGNYTLTLTGGYWAK 139
G2106     GFTPSGTTGTTKLTVTEECQVRVGDLTVAKTRGQLTDAAPIGPVTVQALGCDARQVALKA 60        G2106     TNTPPGNYTLTLTGGYWAK 139
G2102     GFTPSGTTGTTKLTVTEECQVRVGDLTVAKTRGQLTDAAPIGPVTVQALGCDARQVALKA 60        G2102     TNTPPGNYTLTLTGGYWAK 139
G2097     GFTPSGTTGTTKLTVTEECQVRVGDLTVAKTRGQLTDAAPIGPVTVQALGCDARQVALKA 60        G2097     TNTPPGNYTLTLTGGYWAK 139
G2096     GFTPSGTTGTTKLTVTEECQVRVGDLTVAKTRGQLTDAAPIGPVTVQALGCDARQVALKA 60        G2096     TNTPPGNYTLTLTGGYWAK 139
DraE      GFTPSGTTGTTKLTVTEECQVRVGDLTVAKTRGQLTDAAPIGPVTVQALGCDARQVALKA 60        DraE      TNTPPGNYTLTLTGGYWAK 139
G2099     GFTPSGTTGTTKLTVTEECQVRVGDLTVAKTRGQLTDAAPIGPVTVQALGCDARQVALKA 60        G2099     TNTPPGNYTLTLTGGYWAK 139
G2100     GFTPSGTTGTTKLTVTEECQVRVGDLTVAKTRGQLTDAAPIGPVTVQALGCNARQVALKA 60        G2100     TNTPPGNYTLTLTGGYWAK 139
SM252     GFTPSGTTGTTKLTVTEECQVRVGDLTVAKTRGQLTDAAPIGPVTVQALGCDARQVALKA 60        SM252     TNTPPGNYTLTLTGGYWAK 139
JJB17     GFTPSGTTGTTKLTVTEECQVRVGDLTVAKTRGQLTDAAPIGPVTVQALGCDARQVALKA 60        JJB17     TNTPPGNYTLTLTGGYWAK 139
SM293     GFTPSGTTGTTKLTVTEECQVRVGDLTVAKTRGQLTDAAPIGPVTVQALGCDARQVALKA 60        SM293     TNTPPGNYTLTLTGGYWAK 139
G2076     GFTPSGTTGTTKLTVTEECQVRVGDLTVAKTRGQLTDAAPIGPVTVQALGCDARQVALKA 60        G2076     TNTPPGNYTLTLTGGYWAK 139
SM513     GFTPSGTTGTTKLTVTEECQVRVGDLTVAKTRGQLTDAAPIGPVTVQALGCDARQVALKA 60        SM513     TNTPPGNYTLTLTGGYWAK 139
AfaE-III  GFTPSGTTGTTKLTVTEECQVRVGDLTVAKTRGQLTDAAPIGPVTVQALGCNARQVALKA 60        AfaE-III  TNTPPGNYTLTLTGGYWAK 139
          ********************************************************:********                ********** ********

G2152     DTDNFEQGKFFLISDNNRDKLYVNMRPTDNSAWTTDNGVFYKNDVGSWGGIIGIYVDGQQ 120
SM297     DTDNFEQGKFFLISDNNRDKLYVNMRPTDNSAWTTDNGVFYKNDVGSWGGIIGIYVDGQQ 120
JJB30     DTDNFEQGKFFLISDNNRDKLYVNIRPTDNSAWTTDNGVFYKNDVGSWGGIIGIYVDGQQ 120
SM437     DTDNFEQGKFFLISDNNRDKLYVNIRPTDNSAWTTDNGVFYKNDVGSWGGIIGIYVDGQQ 120
SM249     DTDNFEQGKFFLISDNNRDKLYVNIRPTDNSAWTTDNGVFYKNDVGSWGGIIGIYVDGQQ 120
SM246     DTDNFEQGKFFLISDNNRDKLYVNIRPTDNSAWTTDNGVFYKNDVGSWGGIIGIYVDGQQ 120
SM245     DTDNFEQGKFFLISDNNRDKLYVNIRPTDNSAWTTDNGVFYKNDVGSWGGIIGIYVDGQQ 120
SM54      DTDNFEQGKFFLISDNNRDKLYVNIRPTDNSAWTTDNGVFYKNDVGSWGGIIGIYVDGQQ 120
G2171     DTDNFEQGKFFLISDNNRDKLYVNIRPTDNSAWTTDNGVFYKNDVGSWGGIIGIYVDGQQ 120
G2166     DTDNFEQGKFFLISDNNRDKLYVNIRPTDNSAWTTDNGVFYKNDVGSWGGIIGIYVDGQQ 120
G2106     DTDNFEQGKFFLISDNNRDKLYVNIRPTDNSAWTTDNGVFYKNDVGSWGGIIGIYVDGQQ 120
G2102     DTDNFEQGKFFLISDNNRDKLYVNIRPTDNSAWTTDNGVFYKNDVGSWGGIIGIYVDGQQ 120
G2097     DTDNFEQGKFFLISDNNRDKLYVNIRPTDNSAWTTDNGVFYKNDVGSWGGIIGIYVDGQQ 120
G2096     DTDNFEQGKFFLISDNNRDKLYVNIRPTDNSAWTTDNGVFYKNDVGSWGGIIGIYVDGQQ 120
DraE      DTDNFEQGKFFLISDNNRDKLYVNIRPTDNSAWTTDNGVFYKNDVGSWGGIIGIYVDGQQ 120
G2099     GTDNFEQGKFFLISDNNRDKLYVNIRPTDNSAWTTDNGVFYKNDVGSWGGTIGIYVDGQQ 120
G2100     GTDNFEQGKFFLISDNNRDKLYVNIRPTDNSAWTTDNGVFYKNDVGSWGGIIGIYVDGQQ 120
SM252     DTDNFEQGKFFLISDNNRDKLYVNIRPTDNSTWTTDNGVFYKNDVGSWGGVIGIYVDGQQ 120
JJB17     DTDNFEQGKFFLISDNNRDKLYVNIRPTDNSAWTTDNGVFYKNDVGSWGGTIGIYVDGQQ 120
SM293     DTDNFEQGKFFLISDNNRDKLYVNIRPADNSAWTTDNGVFYKNDVGSWGGIIGIYVDGQQ 120
G2076     DTDNFEQDKFFLISDNNRDKLYVNIRPMDNSAWTTDNGVFYKNDVGSWGGIIGIYVDGQQ 120
SM513     DTDNFEQGKFFLISDNNRDKLYVNIRPMDNSAWTTDNGVFYKNDVGSWGGIIGIYVDGQQ 120
AfaE-III  DTDNFEQGKFFLISDNNRDKLYVNIRPMDNSAWTTDNGVFYKNDVGSWGGTIGIYVDGQQ 120
          .******.********************:** ***:************************ ********
```

Abb.4

GFTPSGTTGTTKLTVTEECQVRVGDLTVAKTRGQLTDAAPIGPVTVQALGCXARQVALKAXT
DNFEQXKFFLISDNKRDKLYVNXRPXDNSXWTTDNGVFYKNDVGSWGGXIGIYVDGQQTNTP
PGNYTLXLTGGYWAK

Abb.5, SEQ ID No3

GFTPSGTTGTTKLTVTEECQVRVGDLTVAKTRGQLTDAAPIGPVTVQALGCXARQVALKADT
DNFEQGKFFLISDNNRDKLYVNIRPXDNSAWTTDNGVFYKNDVGSWGGXIGIYVDGQQTNTP
PGNYTLTLTGGYWAK

Abb. 6
SEQ ID No 4

```
XXXXXXXPXXXAEGKXVLLXXXNXXXXXXXYXWXKGXXXXXXXXXIXGYXXXX-
QXXXPGXAXXXREXXYXNXXLLXXNXXXXDXGXYTLXXIXXXXXXXXXTXQXXVXXXXXXPX
XXXXXXXXXXXXXXVXXXXXXXXXXXXXLXXXXXXSXXXXXXXXXXXXXXXXXXXXXXXXXX
XXXXXXXXXXXXAXXXXXXXXXXXYXX
```

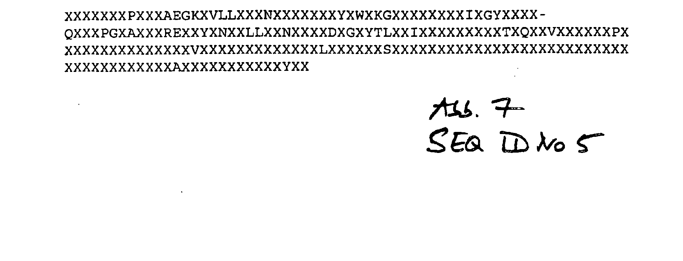

Abb. 7
SEQ ID No 5

Fig. 8

```
CEACAM5 CEA  MESPSAPPHRWCIPWQRLLLTASLLTFWNPPTTAKLTIESTPFNVAEGKEVLLLVHNLPQ 60
CEACAM6 NCA  MGPPSAPPCRLHVPWKEVLLTASLLTFWNPPTTAKLTIESTPFNVAEGKEVLLLAHNLPQ 60
CEACAM8      MGPISAPSCRWRIPWQGLLLTASLFTFWNPPTTAQLTIEAVPSNAAEGKEVLLLVHNLPQ 60
CEACAM7      MGSPSACPYRVCIPWQGLLLTASLLTFWNLPNSAQTNIDVVPFNVAEGKEVLLLVHNWESQ 60
CEACAM1      MGHLSAPLHRVRVPWQGLLLTASLLTFWNPPTTAQLTIESNPFNVAEGKEVLLLVHNLPQ 60
CEACAM3      MGPPSASPHRECIPWQGLLLTASLLNFWNPPTTAKLTIESNPLSVAEGKEVLLLVHNLPQ 60
CEACAM4      MGPPSAAPRGGHRPWQGLLITASLLTFWHPPTTVQFTIEALPSSAAEGKDVLLLACNISE 60
             *   **      **:  :*:;****:.**: *.::  . :  * ..****:***:.  * .:

CEACAM5      HLFGYSWYKGERVDGNRQIIGYVIGT-QQATPGPAYSGREIIYPNASLLIQNIIQNDTGF 119
CEACAM6      NRIGYSWYKGERVDGNSLIVGYVIGT-QQATPGPAYSGRETIYPNASLLIQNVTQNDTGF 119
CEACAM8      DPRGYNWYKGETVDANRRIIGYVISN-QQITPGPAYSNRETIYPNASLLMRNVTRNDTGS 119
CEACAM7      NLYGYNWYKGERVHANYRIIGYVKNISQENAPGPAHNGRETIYPNGTLLIQNVTHNDAGF 120
CEACAM1      QLFGYSWYKGERVDGNRQIVGYAIGT-QQATPGPANSGRETIYPNASLLIQNVTQNDTGF 119
CEACAM3      HLFGYSWYKGERVDGNSLIVGYVIGT-QQATPGAAYSGRETIYTNASLLIQNVTQNDIGF 119
CEACAM4      TIQAYYWHKGKTAEGSPLIAGYITDI-QANIPGAAYSGRETVYPNGSLLFQNITLEDAGS 119
             * *;:**  ,,,, * **  .*  **,* .** ;*.*,:**;;*:   ;* *

CEACAM5      YTLHVIKSDLVNEEATGQFRVYPELPKPSISSNNSKPVEDKDAVAFTCEPETQDATYLWW 179
CEACAM6      YTLQVIKSDLVNEEATGQFHVYPELPKPSISSNNSNPVEDKDAVAFTCEPEVONTTYLWW 179
CEACAM8      YTLQVIKLNLHSEEVTGQFSVHPETPKPSISSNNSNPVEDKDAVAFTCEPETQNTTYLWW 179
CEACAM7      YTLHVIKENLVNEEVTRQFYVFSEPPKPSITSNNFNPVENKDIVVLTCQPETQNTTYLWW 180
CEACAM1      YTLQVIKSDLVNEEATGQFHVYPELPKPSISSNNSNPVEDKDAVAFTCEPETQDTTYLWW 179
CEACAM3      YTLQVIKSDLVNEEATGQFHVQEN-APGLPVGAVAGIVTGVLVGVALVAALVCFLLLAK 178
CEACAM4      YTLRTINASYDSDQATGQLRVHQNN-VPGLPVGAVAGIVTGVLVGVALVAALVCFLLLSR 178
             ***:.*:.  .::.* *: *.: *...:   :    *..  *

CEACAM5      VENQSLPVSPRLQLSNGNRTLTLFNVTRNDTASYKCETQNPVSARRSDSVILNVLYGPDA 239
CEACAM6      VEGQSLPVSPRLQLSNGNRTLTLSVKRNDAGSYECEIQNPASANRSDPVTLNVLYGPDV 239
CEACAM8      VNGQSLPVSPRLQLSNGNRTLTLLSVTRNDVGPYECEIQNPASANFSDPVTLNVLYGPDA 239
CEACAM7      VNNQSLLVSPRLLLSTDNRTLVLLSATKNDIGPYECEIQNPVGASRSDPVTLNVRYES-- 238
CEACAM1      INNQSLPVSPRLQLSNGNRTLTLLSVTRNDTGPYECEIQNPVSANRSDPVTLNVTYGPDT 239
CEACAM3      TGRTSIQRDLREOQP----QALAPGRGPSHSSAFSHSPLSSAQAPLPNPRTAASIYEE-- 232
CEACAM4      TGRASIQRDLREQPP----PASTPGHGPSHRSTFS--------APLPSPRTATPIYEE-- 224
             .  *:  .  .  ..:.  .  * .. .   *

CEACAM5      PTISPLNTSYRSGENLNLSCHAASNPPAQYSWFVNGTFQQSTOELFIPNITVNNSGSYTC 299
CEACAM6      PTISPSKANYRPGENLNLSCHAASNPPAQYSWFINGTFQQSTOELFIPNITVNNSGSYNC 299
CEACAM8      PTISPSDTYRAGVNLNLSCHAASNPPSQYSWSVNGTFQQTTQKLFIPNITTKNSGSYAC 299
CEACAM7      -------------------------------------------------------------
CEACAM1      PTISPSDTYYRPGANLSLSCYAASNPPAQYSWLINGTFQQSTOELFIPNITVNNSGSYTC 299
CEACAM3      -------------------------------------------------------------
CEACAM4      -------------------------------------------------------------

CEACAM5      QAHNSDTGLNRTTVTTITVYAEPPKPFITSNNSNPVEDEDAVALTCEPEIQNTTYLWWVN 359
CEACAM6      QAHNSATGLNRTTVTHITV--------------------------------------- 318
CEACAM8      HTTNSATGRNRTTVRHITVS-------------------------------------- 319
CEACAM7      -------------------------------------------------------------
CEACAM1      HANNSVTGCNRTTVKTIIYTELSP----------------------------------- 323
CEACAM3      -------------------------------------------------------------
CEACAM4      -------------------------------------------------------------

CEACAM5      NQSLPVSPRLQLSNDNRTLTLLSVTRNDVGPYECGIQNELSVDHSDPVILNVLYGPDDPT 419
CEACAM6      -------------------------------------------------------------
CEACAM8      -------------------------------------------------------------
CEACAM7      -------------------------------------------------------------
CEACAM1      -------------------------------------------------------------
CEACAM3      -------------------------------------------------------------
CEACAM4      -------------------------------------------------------------
```

```
CEACAM5   ISPSYTYYRPGVWLSLSCHAASNPPAQYSWLIDGNIQQHTQELFISNITEKWSGLYTCQA 479
CEACAM6   -------------------------------------------------------------
CEACAM8   -------------------------------------------------------------
CEACAM7   -------------------------------------------------------------
CEACAM1   -------------------------------------------------------------
CEACAM3   -------------------------------------------------------------
CEACAM4   -------------------------------------------------------------

CEACAM5   NNSASGHSRTTVKTITVSAELPKPSISSNNSKPVEDKDAVAFTCEPEAQNTTYLWWVNCQ 539
CEACAM6   -------------------------------------------------------------
CEACAM8   -------------------------------------------------------------
CEACAM7   -------------------------------------------------------------
CEACAM1   ------------------VVAKPQIKASKTTVTGDKDSVNLTCSTNDTGISIRWFFKNQ 364
CEACAM3   -------------------------------------------------------------
CEACAM4   -------------------------------------------------------------

CEACAM5   SLPVSPRLQLSNGNRTLTLFNVTRNDARATVCGIQNSVSANRSDPVTLDVLYGPDTPIIS 599
CEACAM6   -------------------------------------------------------------
CEACAM8   -------------------------------------------------------------
CEACAM7   -------------------------------------------------------------
CEACAM1   SLPSSERHKLSQGNTTLSINPVKREDAGTYWCEVFNPISKNQSDPINLU---------- 413
CEACAM3   -------------------------------------------------------------
CEACAM4   -------------------------------------------------------------

CEACAM5   PPDSSYLSGANLNLSCHSASNPSPQYSWRINGIPQQHTQVLFIAKITPNNNGTYACFVSN 659
CEACAM6   -------------------------------------------------------------
CEACAM8   -------------------------------------------------------------
CEACAM7   -------------------------------------------------------------
CEACAM1   -------------------------------------------------------------
CEACAM3   -------------------------------------------------------------
CEACAM4   -------------------------------------------------------------

CEACAM5   LATGRNNSIVKSITVSASGTSPGLSAGATVGIMIGVLVGVALI---------------- 702
CEACAM6   ----------------SGSAPVLSAVATVGITIGVLARVALI---------------- 344
CEACAM8   -------------DALVQGSSPGLSARATVSIMIGVLARVALI--------------- 349
CEACAM7   ----------------VQASSPDLSAGTAVSIHIGVLAGMALI--------------- 265
CEACAM1   -----------VNYNALPQENGLSPGAIAGIVIGVVALVALIAVALACFLHFGKTGRAS 461
CEACAM3   -----------------LLKHDTNIYCRFDHKAEVAS----------------- 252
CEACAM4   ---------------------LLYSDANIYCQIDHRADVVS----------------- 244
          * :   :. . :.

CEACAM5   -------------------------------------------------------------
CEACAM6   -------------------------------------------------------------
CEACAM8   -------------------------------------------------------------
CEACAM7   -------------------------------------------------------------
CEACAM1   DQRDLTEHKPSVSNHTQDHSNDPPEKHNEVTTSTLNFEAQQPTQPTSASPSLTATEIIYS 521
CEACAM3   -------------------------------------------------------------
CEACAM4   -------------------------------------------------------------

CEACAM5   -----
CEACAM6   -----
CEACAM8   -----
CEACAM7   -----
CEACAM1   EVKKQ 526
CEACAM3   -----
CEACAM4   -----
```

CLUSTAL 2.0.8 multiple sequence alignment von CEACAM 1, 3, 4, 5, 6, 7, 8

N-terminale Domäne ist unterstrichen

```
KLTIESTPFNVAEGKEVLLLXHNLPQXXXGYSWYKGERVDGNXXIXGYVIGTQQATPGPAYS
GREXIYPNASLLIQNXXQNDTGFYTLXVIKSDLVNEEATGQFXVYPELPKPSISSNNSXPVE
DKDAVAFTCEPEXQXXTYLWWVNXQSLPVSPRLQLSNGNXTLTLXXVXRNDXXSYXCEXQNP
XSAXRSDXVXLNVLYGPDXPTISPXXXXYRXGENLNLSCHAASNPPAQYSWFXNGTFQQSTQ
ELFIPNITVNNSGSYXCQAHNSXTGLNRTTVTXITVYAEPPKPFITSNNSNPVEDEDAVALT
CEPEIQNTTYLWWVNNAHNSATGLNRTTVTMITVQSLPVSPRLQLSNDNRTLTLLSVTRNDV
GPYECGIQNELSVDHSDPVILNVLYGPDDPTISPSYTYYRPGVNLSLSCHAASNPPAQYSWL
IDGNIQQHTQELFISNITEKNSGLYTCQANNSASGHSRTTVKTITVSAELPKPSISSNNSKP
VEDKDAVAFTCEPEAQNTTYLWWVNGQSLPVSPRLQLSNGNRTLTLFNVTRNDARAYVCGIQ
NSVSANRSDPVTLDVLYGPDTPIISPPDSSYLSGANLNLSCHSASNPSPQYSWRINGIPQQH
TQVLFIAKITPNNNGTYACFVSNLATGRNNSIVKSITVSASGXXPXLSAXATVGIXIGVLXX
VALI
```

Ab. 9
SEQ ID No 6

KLTIESTPFNVAEGKEVLLLVHNLPQHLFGYSWYKGERVDGNRQIIGYVIGTQQATPGPAYS
GREIIYPNASLLIQNIIQNDTGFYTLHVIKSDLVNEEATGQFRVYPELPKPSISSNNSKPVE
DKDAVAFTCEPETQDATYLWWVNNQSLPVSPRLQLSNGNRTLTLFNVTRNDTASYKCETQNP
VSARRSDSVILNVLYGPDAPTISPLNTSYRSGENLNLSCHAASNPPAQYSWFVNGTFQQSTQ
ELFIPNITVNNSGSYTCQAHNSDTGLNRTTVTTITVYAEPPKPFITSNNSNPVEDEDAVALT
CEPEIQNTTYLWWVNNQSLPVSPRLQLSNDNRTLTLLSVTRNDVGPYECGIQNELSVDHSDP
VILNVLYGPDDPTISPSYTYYRPGVNLSLSCHAASNPPAQYSWLIDGNIQQHTQELFISNIT
EKNSGLYTCQANNSASGHSRTTVKTITVSAELPKPSISSNNSKPVEDKDAVAFTCEPEAQNT
TYLWWVNGQSLPVSPRLQLSNGNRTLTLFNVTRNDARAYVCGIQNSVSANRSDPVTLDVLYG
PDTPIISPPDSSYLSGANLNLSCHSASNPSPQYSWRINGIPQQHTQVLFIAKITPNNNGTYA
CFVSNLATGRNNSIVKSITVSASGTSPGLSAGATVGIMIGVLVGVALI

Abb. 10
SEQ ID No. 7

KLTIESTPFNVAEGKEVLLLAHNLPQNRIGYSWYKGERVDGNSLIVGYVIGTQQATPGPAYS
GRETIYPNASLLIQNVTQNDTGFYTLQVIKSDLVNEEATGQFHVYPELPKPSISSNNSNPVE
DKDAVAFTCEPEVQNTTYLWWVNGQSLPVSPRLQLSNGNMTLTLLSVKRNDAGSYECEIQNP
ASANRSDPVTLNVLYGPDVPTISPSKANYRPGENLNLSCHAASNPPAQYSWFINGTFQQSTQ
ELFIPNITVNNSGSYMCQAHNSATGLNRTTVTMITVSGSAPVLSAVATVGITIGVLARVALI

*Abb. 11*
*SEQ ID No 8*

```
GFTPSGTTGT  TKLTVTEECQ  VRVGDLTVAK  TRGQLTDAAP  IGPVTVQALG  50
    N           S        T  S KWK   G  D NSQ    V       T
    F           P        A  G RFR   D  G TS             N
    C           K        S  N L S   S  S LD             C
    S           G        N  A V E   N  N M              S
    V           D        K  T Y Q      L G             G
    R           R           K P F      V F             A
    A           N           R N G      T                P
    I           H           H Q L      A
    L           Q           Q   H
    Y                       M   P
                                N
                                T
                                W

CDARQVALKA  DTDNFEQGKF  FLISDNKRDK  LYVNIRPTDN  SAWTTDNGVF  100
    G                               D V M       L       Y
    N                               S   L       M       V
    S                               H           Y
    C                                           F
    P                                           C
    Q                                           W
    Y                                           Q
    H                                           N
    K                                           E
    R                                           S
    T                                           I

YKNDVGSWGG  IIGIYVDGQQ  TNTPPGNYTL  TLTGGYWAK               H           139
    S           C  VTA P
    A           V  LWS S
    T           H  AEL
    R           Y  CV
    M           T
    V           M
    P           F
    N
    E
    Q
    G
    K
    H
```

EP 2 250 190 B1

Abb. 12

```
GFTPSGTTGT  TKLTVTEECQ  VRVGDLTVAK  TRGQLTDAAP  IGPVTVQALG  50
         N          S   T S KWK G  D  NSQ V          T
         F          P   A G RFR D  G   TS           N
         C          K   S N L S S  S   LD           C
         S          G   N A V E N  N    M           S
         V          D   K T Y Q    L    G           G
         R          R     K P F    V    F           A
         A          N     R N G    T                P
         I          H     H Q L    A
         L          Q     Q   H
         Y          M
                        M   P
                            N
                            T
                            W

CDARQVALKA  DTDNFEQGKF  FLISDNKRDK  LYVNIRPTDN  SAWTTDNGVF  100
G                                   D V M          L   Y
N                                   S   L          M   V
S                                   H              Y
C                                                  F
P                                                  C
Q                                                  W
Y                                                  Q
H                                                  N
K                                                  E
R                                                  S
T                                                  I
                                                   H       139
YKNDVGSWGG  IIGIYVDGQQ  TNTPPGNYTL  TLTGGYWAK
         S   C VTA P
         A   V LWS S
         T   H AEL
         R   Y CV
         M   T
         V   M
         P   F
         N
         E
         Q
         G
         K
         H
```

Abb. 13

```
GFTPSGTTGT TKLTVTEECQ VRVGDLTVAK TRGQLTDAAP IGPVTVQALG 50
     N          S      T S KWK    G D NSQ    V    T
     F          P      A G RFR    D G TS          N
     C          K      S N LS     S S LD          C
     S          G      N A VE     N N M           S
     V          D      K T YQ       L G           G
     R          R        K PF       V F           A
     A          N        R NG       T             P
     I          H        H QL       A
     L          Q        Q H
     Y          M        M P
                             N
                             T
                             W

CDARQVALKA DTDNFEQGKF FLISDNKRDK LYVNIRPTDN SAWTTDNGVF 100
 G                                D V M        L Y
 N                                S   L        M V
 S                                H            Y
 C                                             F
 P                                             C
 Q                                             W
 Y                                             Q
 H                                             N
 K                                             E
 R                                             S
 T                                             I
                                               H
YKNDVGSWGG IIGIYVDGQQ TNTPPGNYTL TLTGGYWAK     H       139
     S      C VTA P
     A      V LWS S
     T      H AEL
     R      Y CV
     M      T
     V      M
     P      F
     N
     E
     Q
     G
     K
     H
```

Abb.14

EECQVRVGDLTVAKTRGQLTDAAPIGPVTVQALGCDARQVALKADTDNFEQGKFFLISDNKR
DKLYVNIRPMDNSAWTTDNGVFYKNDVGSWGGVIGVYADGQQTNTPPGNYTLTLTGGYWAKD
NKQGFTPSGTTGTTKLTVT

ASS. 15
SEQ ID 12

Gemittelte Fluoreszenz in Mausorganen
(normiert auf Tag 2)

Abb. 16

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2007057182 A3
- EP 1777292 A1
- US 20040247861 A
- US 6179912 B
- US 7147712 B2
- WO 2005001889 A
- WO 2004003558 A
- WO 200400355 A

- US 20040247861 A1
- EP 1721974 A1

### In der Beschreibung aufgeführte Nicht-Patentliteratur

- **Berger et al.** Differential recognition of members of the carcinoembryonic antigen family by Afa/Dr adhiesins of diffusely adhering Escherichia coli (Afa/Dr DAEC). *Molecular Microbiology,* 2004, vol. 52 (4), 963-983
- **Alain L. Servin.** Pathogenesis of Afa/Dr Diffusely Adhering Escherichia coli. *Clinical Microbiology Reviews,* April 2005, vol. 18 (2), 264-292
- **Piatek et al.** Molecular Aspects of Biogenesis of Escherichia coli Dr Fimbriae: Characterization of DraB-DraE-Complexes. *Infection and Immunity,* 2005, vol. 73 (1), 135-145

- **van Loy et al.** Identification of amino acids in Dr adhesin required for binding to decay-accelerating factor. *Molecular Microbiology,* 2002, vol. 45 (2), 439-452
- **Saturag et al.** *British Journal of Nutrition,* 2000, vol. 84, 791-802
- **Korotkova et al.** A Subfamily of Dr Adhesins of Escherichia coli Bind Independently to Decay-accelerating Factor and the N-domain of Carcinoembryonic Antigen. *The Journal of Biological Chemistry,* 2006, vol. 281 (39), 29120-29139